# EUROPEAN PATENT APPLICATION

(11) **EP 4 843 503 A1**
(43) Date of publication of application: **25.11.2026**
(21) Application number: 26180083.3
(22) Date of filing: 21.05.2026
(51) Int. Cl.: A61B 5/346, A61B 5/00, G06N 3/08, G16H 10/00

(54) **METHOD AND SYSTEM FOR ELIMINATING FAR FIELD (FF) EFFECTS IN UNIPOLAR SIGNALS USING DEEP LEARNING**

(30) Priority: 22.05.2025 US 202519216356
(71) Applicant: Biosense Webster (Israel) Ltd., 2066717 Yokneam (IL)
(72) Inventor: GALUTIN, Arye Leybale, 2066717 Yokneam (IL); GUZI, Eliran, 2066717 Yokneam (IL)
(74) Representative: Carpmaels & Ransford LLP

(57) **Abstract**

A method and system for reducing far-field interference in unipolar cardiac signals using a two-stage deep learning approach are disclosed. In the first stage, a multipolar algorithm is applied to multiple unipolar signals recorded from a cardiac mapping catheter to estimate and remove far-field components, generating a multipolar signal that serves as a ground truth label. In the second stage, a deep learning model is trained using unipolar signals as inputs and the corresponding multipolar signals as labels, enabling the model to extract near-field components while minimizing far-field interference. The trained deep learning model is then deployed to process new unipolar signals, generating an enhanced output signal with improved near-field clarity without requiring additional unipolar inputs. The system supports real-time signal processing and integration with electrophysiology mapping and catheter ablation procedures. The disclosed invention improves the accuracy of cardiac signal interpretation, enhances arrhythmia detection, and facilitates high-precision ablation therapy.

## Description

### FIELD OF THE INVENTION

The present invention relates to the field of cardiac mapping and signal processing. More specifically, it concerns a method and system for eliminating far-field (FF) effects from unipolar signals using a deep learning-based approach.

### BACKGROUND OF THE INVENTION

Cardiac electrophysiology relies on the accurate measurement and analysis of electrical signals to understand the heart's electrical activity and diagnose arrhythmias. Among these measurements, unipolar signals play a crucial role. A unipolar signal is recorded using a single electrode placed in contact with the heart tissue, measuring electrical potential relative to a reference electrode positioned at a distant, electrically neutral site, such as the chest, back, or limb. These signals provide a broad view of cardiac electrical activity, encompassing both local (near-field) and distant (far-field) components. The near-field component reflects the localized electrical activity of myocardial tissue adjacent to the electrode, while the far-field component represents contributions from more distant regions of the heart. The far component may also include noise from nearby machinery such as an MRI. As such, the far-field can be generalized to be contribution to the Unipolar signal which is not reflecting the myocardial tissue adjacent to the electrode

Unipolar signals are valuable for identifying general trends in electrical conduction and characterizing tissue properties, such as detecting areas of low voltage indicative of scarring or fibrosis. However, their broad sensitivity can be a limitation when precise localization of electrical sources is required, such as during the diagnosis or treatment of arrhythmias. This limitation arises because unipolar signals inherently blend near-field and far-field components, making it challenging to isolate the localized electrical activity of interest.

Electrophysiological mapping relies on unipolar signals acquired from catheters to assess cardiac electrical activity. However, unipolar signals are susceptible to far-field interference, which can obscure meaningful local electrical activity. Traditional approaches, such as the Multipolar algorithm, require multiple unipolar signals to estimate and remove far-field interference. These approaches are limited by catheter design, the availability of multiple input signals, and decreased performance in certain arrhythmias, such as atrial fibrillation.

Conventional methods for extracting near-field signals from unipolar recordings include bipolar recordings, filtering, and manual interpretation by electrophysiologists. Bipolar signals, derived from the difference between two closely spaced unipolar electrodes, are effective at suppressing far-field components but can also attenuate near-field signals, resulting in a loss of critical detail. High-pass filtering can isolate higher-frequency near-field components but may inadvertently distort signal fidelity. Manual interpretation is subjective and depends on the expertise of the electrophysiologist, making it prone to variability and unsuitable for large-scale or automated analysis. Moreover, these techniques often struggle to achieve the level of precision and resolution required for modern electrophysiological procedures, such as catheter ablation or complex arrhythmia mapping.

For example, US Patent Publication US20210393187A1 by Amos et al describes a method for improving the accuracy of electrocardiograms (ECGs) by using an autoencoder to process intracardiac signals. This method involves receiving input signals that contain artifacts, encoding these signals to produce a latent representation, and then decoding them to reconstruct the signals without the artifacts.

In addition, WIPO Publication WO2023111798A1 by Rodriguez describes a mapping engine that performs spatial electrode signal analysis to identify and scale common signal components, thereby isolating residual signals.

Likewise, US Patent Publication 20220051091 by Ravnua describes a method for collecting bipolar and unipolar electrograms annotated by human reviewers with a window-of-interest and activation times, generating a ground truth data set from the electrograms. This data set is used to train a machine learning algorithm to preprocess electrograms and detect activation occurrences in a given bipolar electrogram within the window of interest.

Similarly, US Patent Publication US20210169394A1 by Chou et al. describes a processing module that utilizes algorithms and high-performance processors to analyze data from the mapping and imaging subsystems. This module uses machine learning and predictive algorithms to interpret the data, creating functional models of the cardiac anatomy and identifying optimal treatment strategies.

Further, US Patent Publication US 20220175302 by Botzer et al. describes a machine learning/artificial intelligence method for generating electrocardiograms from multiple references. It utilizes an interpretation engine that processes bipolar intracardiac reference signals from catheter electrodes.

The present invention addresses these limitations by introducing a deep learning-based method that processes a single unipolar signal to remove far-field interference, thereby improving signal accuracy and broadening applicability across different catheter designs and electrophysiological conditions. Embodiments of this invention improve upon and surpasses the performance of these conventional systems and methods by integrating multipolar signals, advanced preprocessing, and deep learning to achieve robust and scalable near-field signal extraction. The approach is particularly valuable for applications in cardiac electrophysiology, where accurate localization of electrical activity is critical for diagnosis and treatment. By addressing the limitations of existing methods and harnessing the strengths of multipolar and unipolar signals, the disclosed method offers a significant improvement in the extraction and analysis of near-field components.

### SUMMARY OF THE INVENTION

The present invention relates to a method and system for reducing far-field interference in unipolar cardiac signals using a two-stage deep learning approach. In the first stage, a multipolar algorithm is applied to multiple unipolar signals recorded from a cardiac mapping catheter to estimate and subtract far-field components, thereby generating a multipolar signal that represents near-field activity with reduced interference. These multipolar signals serve as ground truth labels for training a deep learning model. By leveraging multipolar signal processing as a reference, the deep learning model learns to extract near-field components from unipolar signals with high accuracy, overcoming the limitations of traditional signal filtering and manual interpretation methods.

In the second stage, the trained deep learning model is deployed to process new unipolar signals in real time, removing far-field interference without requiring multiple unipolar inputs. This enables single-signal far-field suppression, making the system adaptable to various catheter designs and clinical conditions. The invention improves cardiac mapping precision, enhances arrhythmia detection, and supports high-precision catheter ablation procedures by providing real-time near-field signal clarity. The method is optimized for integration into electrophysiology systems, including electroanatomical mapping platforms and automated arrhythmia detection tools, ensuring broad applicability in cardiac diagnostics and treatment.

### BRIEF DESCRIPTION OF THE DRAWINGS

A more detailed understanding may be had from the following description, given by way of example in conjunction with the accompanying drawings, wherein like reference numerals in the figures indicate like elements.
FIG. 1 is a diagram of an example cardiac ablation system, based on which one or more features of the disclosure may be implemented.
FIG. 2 is a block diagram of an example system, deployable by the example cardiac ablation system of FIG. 1, based on which one or more features of the disclosure may be implemented.
FIG. 3 illustrates a block diagram depicting the two-stage methodology for reducing far-field interference in unipolar cardiac signals using a deep learning-based approach.
FIG. 4 is a diagram illustrating the Multipolar Algorithm.
FIG. 5 illustrates a flowchart of the two-stage method for reducing far-field interference in unipolar cardiac signals using a deep learning-based approach.
FIG. 6 illustrates a flowchart describing the overall workflow of training and deploying a deep learning model for reducing far-field interference in unipolar cardiac signals.
FIG. 7 illustrates a flowchart outlining the method for training and deploying a deep learning model to suppress far-field interference in unipolar cardiac signals.

### DETAILED DESCRIPTION OF THE PREFERRED EMBODIMENTS

In cardiac electrophysiology, unipolar signals recorded from mapping catheters contain both near-field and far-field components, making it challenging to accurately localize arrhythmic sites. Far-field interference, caused by electrical activity from distant cardiac regions or extracardiac sources, obscures near-field signal clarity, complicating electroanatomical mapping, activation time analysis, and voltage-based arrhythmia characterization. Traditional methods for addressing this issue include bipolar recordings, high-pass filtering, and manual interpretation, but these approaches have significant limitations. Bipolar signals, while reducing far-field contributions, also remove valuable near-field information and are highly dependent on electrode spacing and orientation. Filtering techniques are often imprecise, failing to selectively suppress far-field components without distorting near-field signals. Manual interpretation, commonly used in clinical practice, introduces subjectivity and variability, leading to inconsistencies in arrhythmia diagnosis and ablation planning. These challenges create a critical need for an automated, accurate, and real-time method for isolating near-field activity in unipolar signals.

The invention provides a two-stage deep learning-based approach to eliminate far-field interference from unipolar signals while preserving near-field signal integrity. In the first stage, a multipolar algorithm is applied to multiple unipolar signals recorded from an electrophysiology catheter to estimate and remove far-field components, generating a multipolar signal with enhanced near-field clarity. This multipolar signal serves as a ground truth label for training a deep learning model. In the second stage, the deep learning model is trained using unipolar signals as inputs and the multipolar signals as reference labels, enabling it to learn the underlying patterns of far-field suppression. Once trained, the deep learning model can be deployed in real-time to process single unipolar signals, generating a far-field-reduced output without requiring additional input signals. This approach overcomes the limitations of traditional multipolar algorithms by eliminating the need for multiple unipolar signals during inference, allowing for real-time, catheter-independent far-field suppression. The invention enhances cardiac signal clarity, improving the precision of electroanatomical mapping, arrhythmia detection, and catheter ablation guidance, ultimately leading to more effective and reproducible clinical outcomes in electrophysiology procedures.

Unipolar signals in cardiac mapping are electrical signals recorded using a single electrode placed in contact with the heart tissue, measured relative to an indifferent electrode typically located at a distant position on the body (such as the chest or back). These signals provide essential insights into the heart's local and global electrical activity, making them a critical tool in electrophysiological studies and cardiac mapping procedures.

A unipolar signal captures the potential difference between the electrode on the heart and the remote reference electrode. The resulting waveform typically includes components such as the P wave (atrial activity), QRS complex (ventricular depolarization), and T wave (ventricular repolarization). The shape and polarity of these components vary depending on the electrode's position relative to the propagating electrical wavefront. For example, a depolarization wavefront moving toward the electrode produces a negative deflection, while one moving away results in a positive deflection.

Unipolar signals are widely used in cardiac mapping techniques, such as activation mapping and voltage mapping. They offer a global view of the heart's electrical activity, capturing both local signals and those from more distant regions. This broad perspective helps identify general trends and abnormalities in electrical conduction. Unipolar signals are particularly valuable for understanding wavefront direction and tissue characterization, as they can reveal regions of low voltage associated with scarring or fibrosis.

Despite their advantages, unipolar signals have some limitations. Because they capture both local and remote electrical activity, distinguishing between the two can be challenging. They are also more prone to noise from body movement or poor electrode contact, and their broader focus can lack the spatial resolution needed for precise localization of small arrhythmogenic sites. In such cases, bipolar signals, which measure the difference between two adjacent electrodes, provide better spatial specificity.

Unipolar signals are collected during cardiac mapping by recording the electrical activity of the heart using a single electrode placed in direct contact with the myocardium. This electrode measures the electrical potential at a specific location on the heart relative to a reference electrode positioned at a distant, electrically neutral site on the body, such as the chest, back, or limb. The process involves several key steps to ensure accurate and meaningful signal acquisition.

First, a catheter equipped with multiple electrodes is introduced into the heart, typically through a vein or artery, such as the femoral vein. Each electrode on the catheter can serve as a recording site, capturing electrical signals from different regions of the heart. The catheter is guided to specific areas within the heart chambers under fluoroscopic or 3D mapping system guidance to ensure precise electrode placement.

Once in position, the electrode captures the electrical potential generated by cardiac cells as they depolarize and repolarize during each heartbeat. These electrical potentials are transmitted to an electrophysiology recording system, where they are amplified and filtered to remove noise or unwanted signals. The unipolar signal is then displayed as a time-dependent waveform, typically consisting of the P wave, QRS complex, and T wave, which correspond to the electrical activity of the atria and ventricles.

The reference electrode used in conventional unipolar recording is strategically placed far from the heart to minimize its own contribution to the recorded signal. This distant placement allows the reference electrode to maintain a relatively constant electrical potential, ensuring that the recorded signal primarily reflects the local electrical activity at the heart's surface near the active electrode.

Modern cardiac mapping systems often integrate unipolar signals into 3D electroanatomical maps. These systems process the collected signals to generate spatial representations of electrical activation, conduction velocity, and voltage. These maps provide detailed insights into arrhythmogenic regions and the underlying electrical conduction patterns.

The reference electrode in cardiac mapping is essential for collecting unipolar signals, as it provides a stable electrical baseline against which the electrical potential at a specific heart location, measured by the active electrode, is compared. This comparison generates the unipolar signal, which reflects the local electrical activity of the heart relative to the reference point. To ensure stability, the reference electrode is typically positioned far from the heart at a location where electrical potential remains consistent and is unaffected by localized cardiac activity. Common placements include the chest wall, back, or limbs, and in some cases, an indifferent electrode such as the Wilson central terminal is used. The Wilson central terminal combines electrical potentials from three limb leads (right arm, left arm, and left leg) to create a quasi-neutral point, providing a consistent baseline for unipolar recordings.

The reference electrode's distance from the heart is crucial, as it prevents interference from localized electrical activity and ensures the unipolar signal accurately represents the activity at the active electrode. The electrode acts as a "zero potential" point, measuring the potential difference between itself and the active electrode in contact with heart tissue. Any instability or noise at the reference electrode, such as that caused by poor contact or external interference, can introduce distortions or artifacts into the recorded signal, compromising its accuracy. Additionally, while the Wilson central terminal or other reference setups aim to maintain neutrality, factors like abnormal body geometry or improper placement may introduce slight variations.

Despite its importance, the reference electrode has limitations. It may pick up cardiac signals if placed too close to the heart, leading to contamination of the unipolar signal. External noise can also affect its stability, highlighting the need for careful placement and optimal contact. Nevertheless, the reference electrode remains a critical component in conventional cardiac mapping, ensuring the reliable collection of unipolar signals by serving as a consistent and neutral electrical baseline.

Multipolar signals, in the context of cardiac electrophysiology or bioelectric signal processing, refer to electrical signals recorded using an electrode configuration that involves three or more poles or measurement points. These signals are commonly acquired using catheters or electrode arrays with multiple closely spaced electrodes, providing detailed spatial resolution. This capability makes multipolar signals particularly useful in cardiac mapping and other biomedical applications. By utilizing multiple electrodes, multipolar recordings offer a more comprehensive view of electrical activity in a specific region, such as the heart. The signals are typically derived as differences in electrical potential between pairs or groups of electrodes, which helps minimize noise and suppress far-field effects.

In cardiac mapping, multipolar signals enable the creation of high-resolution maps of electrical activity within the heart. Catheters equipped with multipolar electrodes, often arranged in circular, linear, or grid-shaped arrays, are used to capture simultaneous signals from various locations, aiding in diagnosing arrhythmias or conduction abnormalities. These recordings are especially effective at distinguishing near-field (local) signals from far-field (distant) electrical activity, improving diagnostic precision. Furthermore, they are instrumental in electrophysiological studies for analyzing the propagation of action potentials, identifying reentrant circuits, and locating focal sources of arrhythmias.

Multipolar signals enhance spatial and temporal resolution, offering significant advantages over simpler recording methods. They reduce common-mode noise through differential measurements, providing more reliable data. However, this complexity comes with challenges, including the need for precise electrode placement and the management of large volumes of data, which require advanced signal processing techniques.

Multipolar signals and unipolar signals are closely related in the field of electrophysiology, as they both represent electrical potentials recorded by electrodes in biological tissues, such as the heart. Their relationship lies in how the signals are derived, the information they provide, and their applications in analyzing electrical activity.

A unipolar signal is recorded by measuring the electrical potential at a single electrode relative to a distant reference electrode, often placed outside the region of interest (e.g., on the body surface). This type of signal captures the total electrical activity at the electrode's location, including contributions from both local (near-field) and distant (far-field) electrical sources. As a result, unipolar signals provide a broad view of the overall electrical environment but may lack the spatial specificity required to isolate localized activity.

A multipolar signal, on the other hand, is typically derived from multiple unipolar signals. It represents the difference in electrical potential between two or more electrodes within a multipolar electrode array. For example, a bipolar signal (a simple form of a multipolar signal) is calculated as the difference between the potentials recorded by two adjacent electrodes. By comparing the signals between closely spaced electrodes, multipolar signals emphasize local electrical activity (near-field signals) and reduce the influence of far-field contributions. This makes multipolar signals more sensitive to spatial variations in electrical activity, providing higher spatial resolution compared to unipolar signals.

The relationship between these signals is particularly evident in how they complement each other. Unipolar signals are valuable for assessing global patterns of electrical activity and identifying large-scale phenomena, while multipolar signals are better suited for detailed analysis of localized events, such as wavefront direction and conduction velocity. In cardiac mapping, both signal types are often used in tandem: unipolar signals help provide a broad overview of the heart's electrical activity, while multipolar signals offer the precision needed to pinpoint arrhythmogenic sites or reentrant circuits.

In essence, multipolar signals are derived from unipolar signals and serve as a refined version with enhanced spatial resolution and noise reduction. Together, they form a comprehensive framework for analyzing complex electrical phenomena in biological tissues.

A near-field signal in the context of cardiac mapping refers to the electrical activity recorded by an electrode that is in close proximity to the source of the signal, such as the myocardial tissue. Near-field signals are highly localized and represent the electrical potential generated directly by the depolarization and repolarization of nearby cardiac cells. These signals are distinct from far-field signals, which are electrical signals originating from distant regions of the heart and are more diffuse in nature.

Near-field signals are characterized by their sharp, high-amplitude waveforms. This is because the electrode is close enough to detect the rapid changes in electrical potential associated with the direct activation of myocardial tissue. These signals provide precise information about the timing and location of local electrical events, such as the depolarization of a specific region of the heart. The high resolution of near-field signals makes them invaluable for detailed cardiac mapping, particularly for identifying arrhythmogenic foci or conduction pathways.

In electrophysiological studies, near-field signals are often used to create activation maps, which show the sequence of electrical activity across the heart. For example, during catheter ablation, near-field signals help locate the precise site of an arrhythmia's origin, guiding the ablation procedure. The distinction between near-field and far-field signals is crucial in this context, as far-field signals could obscure the localized activity and complicate the interpretation of the mapping data.

The quality of near-field signals depends on the proximity of the recording electrode to the myocardial tissue. Electrodes placed closer to the tissue surface will capture clearer, more localized near-field signals, whereas those further away may also record far-field activity. Near-field signals are particularly important in procedures requiring high spatial resolution, such as those involving complex arrhythmias like atrial fibrillation or ventricular tachycardia, where understanding the precise electrical activation patterns is critical for effective treatment.

Far-field signals in cardiac mapping refer to electrical signals recorded by an electrode that originate from distant regions of the heart, rather than from the local myocardial tissue near the electrode. These signals are broader and lower in amplitude compared to near-field signals because the source of the electrical activity is further away and is subject to attenuation and dispersion as it travels through the body's tissues.

Far-field signals represent the summation of electrical activity from a larger or more distant region of the heart, such as an adjacent cardiac chamber. For example, an electrode placed in the right atrium may pick up far-field signals from the left atrium or the ventricles. These signals can provide a more generalized view of cardiac electrical activity, but they lack the spatial specificity of near-field signals. The morphology of far-field signals tends to be smoother and less sharp because they are not directly influenced by the rapid local depolarization and repolarization of nearby cells.

In electrophysiology, distinguishing far-field signals from near-field signals is essential for accurate interpretation of mapping data. Far-field signals can sometimes obscure or overlap with near-field signals, making it challenging to pinpoint the exact site of an arrhythmia or conduction pathway. Advanced filtering techniques and careful electrode placement are often employed to minimize the influence of far-field signals when focusing on localized activity.

Far-field signals are often considered less valuable for precise diagnostic or therapeutic procedures, such as catheter ablation, which require detailed information about local electrical activity. However, they can still provide useful context about the overall electrical activity of the heart and may help identify large-scale conduction abnormalities or confirm the presence of synchronized activity across different regions of the heart. Understanding and managing far-field signals is a critical aspect of cardiac mapping and electrophysiological studies, ensuring accurate representation of both local and global electrical behavior in the heart.

Unipolar, far-field, and near-field signals are closely interconnected in cardiac electrophysiology, as they all contribute to understanding the heart's electrical activity. A unipolar signal is recorded by an electrode placed on the heart tissue, and electrical potential is measured relative to a distant reference electrode. This signal captures the total electrical activity at the recording site, combining both near-field and far-field components. Near-field signals represent the local electrical activity of myocardial tissue directly adjacent to the electrode and are characterized by sharp, high-amplitude deflections in the waveform. These signals provide precise, localized information about the timing and sequence of depolarization or repolarization events at the electrode's position. In contrast, far-field signals originate from distant regions of the heart and are smoother, lower in amplitude, and more diffuse due to attenuation as they travel through body tissues. Far-field signals reflect the broader electrical activity of the heart but may overlap or interfere with near-field signals, complicating the interpretation of unipolar recordings.

Unipolar signals inherently blend these two components, with the relative contribution of near-field and far-field signals depending on factors such as the electrode's proximity to the active myocardial tissue and the strength of distant electrical sources. Electrodes positioned closer to the heart capture stronger near-field signals, while far-field signals may dominate in cases where distant regions, such as the ventricles, generate synchronized or large-scale electrical activity. Understanding this relationship is critical in cardiac mapping, where near-field signals are essential for pinpointing the origin of arrhythmias, while far-field signals provide context about the heart's overall electrical activity. Advanced mapping systems often employ algorithms to separate near-field and far-field contributions within unipolar recordings, enabling clearer identification of arrhythmogenic sites. This interplay highlights the importance of unipolar signals in providing both localized and global insights into cardiac function.

Near-field signals are conventionally extracted from a unipolar signal by isolating the local electrical activity originating directly beneath or near the recording electrode. Since unipolar signals inherently include both near-field and far-field components, several techniques are employed to separate the near-field signals from the broader far-field contributions. These methods focus on amplifying the localized, high-amplitude features of the near-field signal while minimizing or filtering out the diffuse, lower-amplitude far-field activity.

One common approach is spatial filtering using bipolar recordings. In this method, the difference in potential between two closely spaced electrodes (on the same catheter) is measured. Bipolar signals emphasize the differences in electrical activity between the electrodes, which tend to capture the localized near-field signal and cancel out far-field contributions that are similar across both electrodes. By subtracting the signals from the two electrodes, the resulting bipolar signal suppresses the far-field components because these are relatively uniform across the small inter-electrode distance.

Another method involves using signal processing techniques, such as high-pass filtering, to remove the lower-frequency components of the unipolar signal, which are often associated with far-field activity. Near-field signals, being sharper and higher in amplitude, contain higher frequency components compared to the broader far-field signals. Applying a high-pass filter can help retain the near-field signal while reducing far-field contributions.

Modern cardiac mapping systems employ advanced signal decomposition algorithms to separate near-field and far-field signals. Advanced signal decomposition algorithms are computational methods used to analyze complex signals by breaking them down into their constituent components. In cardiac electrophysiology, these algorithms are essential for separating near-field signals, which represent localized electrical activity, from far-field signals that originate from distant regions of the heart. Techniques such as Fourier Transform and spectral analysis help differentiate these signals by isolating highfrequency components associated with near-field signals from the lower-frequency, smoother far-field signals. Wavelet Transform, another key approach, excels in capturing transient features like sharp deflections in near-field signals, making it ideal for analyzing dynamic cardiac events. Statistical methods like Independent Component Analysis (ICA) and Principal Component Analysis (PCA) identify unique patterns or dominant components in the data to isolate localized activity, while Empirical Mode Decomposition (EMD) decomposes signals into oscillatory components, distinguishing near-field features from broader far-field oscillations.

In some cases, electrophysiologists may manually interpret the signals to identify near-field activity, relying on their expertise to distinguish sharp, high-amplitude deflections (indicative of near-field signals) from broader, smoother waveforms (far-field signals). The effectiveness of these methods depends on the placement of the electrode, the proximity to the myocardium, and the characteristics of the arrhythmia being studied.

Conventional near-field signal extraction techniques in cardiac mapping, such as bipolar recordings, high-pass filtering, and manual interpretation, have several limitations that can impact the accuracy and effectiveness of isolating near-field signals from unipolar recordings. These limitations arise from the inherent complexity of cardiac signals and the overlapping nature of near-field and far-field components.

One major limitation is the incomplete suppression of far-field signals. Techniques like bipolar recording, which rely on measuring the potential difference between two closely spaced electrodes, reduce far-field signals that are uniform across the electrodes. However, this suppression is not always complete, especially when far-field signals are strong or originate from regions with significant synchronized electrical activity, such as ventricular depolarization. In such cases, far-field signals can still dominate the bipolar recording and obscure near-field components.

Loss of signal detail is another challenge. Bipolar recordings focus on the difference between two electrodes, which can filter out valuable near-field information that does not exhibit significant variation across the electrodes. This can lead to a reduction in the amplitude or sharpness of near-field signals, potentially making them less detectable or interpretable. Similarly, high-pass filtering, while effective at reducing far-field contributions, can inadvertently remove low-frequency components of near-field signals, leading to distortion or loss of signal fidelity.

Manual interpretation of signals, often used to identify near-field activity, is highly dependent on the expertise of the electrophysiologist. This approach is subjective and prone to variability, as distinguishing near-field signals from far-field components based on waveform morphology and amplitude alone can be challenging. Additionally, manual analysis can be time-consuming and impractical for complex or large-scale mapping procedures.

Another limitation is the sensitivity to electrode placement. Near-field extraction techniques are highly dependent on the proximity of the recording electrodes to the myocardial tissue. If the electrodes are not optimally positioned, the near-field signals may be weak or mixed with stronger far-field activity, reducing the effectiveness of conventional extraction methods. Variations in anatomy and electrode alignment can further complicate the isolation process.

Finally, inadequate noise filtering poses a challenge in conventional techniques. Background noise from sources such as muscle activity, patient movement, or equipment interference can overlap with the near-field signals, complicating their isolation. Conventional filtering methods often struggle to differentiate between noise and legitimate far-field or near-field components, leading to potential inaccuracies in signal extraction.

Accordingly, while conventional near-field extraction techniques provide valuable insights, their limitations include incomplete far-field suppression, loss of signal detail, reliance on manual interpretation, sensitivity to electrode placement, and inadequate noise filtering. These challenges highlight the need for more advanced signal processing techniques, such as machine learning and signal decomposition algorithms, to improve the accuracy and reliability of near-field signal extraction in cardiac mapping.

Machine learning (ML) is a branch of artificial intelligence (AI) that enables systems to learn from data and improve their performance on specific tasks without explicit programming. Instead of relying on predefined rules, machine learning algorithms analyze data using statistical techniques to identify patterns, make predictions, and adapt to new information. The process begins with data collection, where large volumes of structured or unstructured data are gathered. This data is then preprocessed, which involves cleaning, normalizing, and transforming it into a format suitable for analysis. During training, a machine learning algorithm is fed a subset of the data, called a training dataset, to learn relationships between input features and desired outputs. The algorithm's performance is fine-tuned using a validation dataset, and its accuracy is ultimately tested on a separate test dataset to ensure it can generalize to new data. Once trained, the model is deployed to make predictions or automate decision-making.

There are several types of machine learning, including supervised learning, where algorithms learn from labeled data with known outcomes; unsupervised learning, which discovers patterns in unlabeled data; semi-supervised learning, which combines labeled and unlabeled data; and reinforcement learning, where an algorithm learns through interaction with an environment, receiving feedback in the form of rewards or penalties. In some instance, self-supervised learning may be utilized.

Core components of machine learning include the algorithms used to process data, features that represent input variables, and the model that results from the learning process. Common algorithms include decision trees, support vector machines, and neural networks, each tailored to specific tasks such as regression, classification, or clustering.

Supervised learning is a type of machine learning where the algorithm is trained using labeled data. In this context, "labeled data" refers to a dataset where each input is paired with a corresponding correct output, often referred to as the "label." The goal of supervised learning is for the algorithm to learn a mapping function that relates the inputs to the outputs, so it can make accurate predictions or classifications when presented with new, unseen data.

The supervised learning process begins with a training dataset, which consists of input-output pairs. For example, in a dataset used to predict house prices, the inputs might include features such as square footage, number of bedrooms, and location, while the output would be the actual price of the house. The algorithm analyzes these examples during the training phase, adjusting its internal parameters to minimize the difference between its predictions and the actual outputs. This process is often guided by a loss function, which quantifies the error in the predictions.

A loss function in machine learning is a mathematical tool used to measure the difference between the predicted output of a model and the actual target value. It quantifies how well or poorly the model is performing, providing a basis for improvement during the training process. When a model makes a prediction, the loss function calculates the error by comparing the predicted value with the true value. This error guides the optimization process, helping the model adjust its internal parameters-such as weights and biases in a neural network-to minimize future errors. The overall goal of training is to minimize the loss function, which in turn enhances the accuracy and reliability of the model's predictions.

Different types of loss functions are used depending on the type of task, such as regression or classification. For regression tasks, loss functions like Mean Squared Error (MSE) and Mean Absolute Error (MAE) are common. MSE calculates the average of the squared differences between predicted and actual values, penalizing larger errors more heavily, while MAE computes the average of the absolute differences, making it less sensitive to outliers. For classification tasks, loss functions like Cross-Entropy Loss and Hinge Loss are widely used. Cross-Entropy Loss measures the difference between the predicted probability distribution and the true class labels, often used in both binary and multi-class classification problems. Hinge Loss, on the other hand, is employed in Support Vector Machines to ensure a margin of separation between different classes.

The loss function plays a central role in training by guiding optimization algorithms, such as gradient descent, which adjust the model's parameters based on the error calculated. This involves computing the gradient of the loss function with respect to the model's parameters and updating these parameters to reduce the loss in subsequent iterations. The choice of loss function is crucial, as it directly affects the model's performance. For instance, while MSE is effective for regression tasks, it is unsuitable for classification. Additionally, some loss functions, such as MSE, are sensitive to outliers as they penalize large errors more heavily, whereas MAE provides greater robustness in such cases.

There are two main types of supervised learning tasks: regression and classification. Regression involves predicting a continuous value, such as forecasting stock prices or estimating the temperature. Classification, on the other hand, involves categorizing data into discrete classes, such as identifying whether an email is spam or not, or recognizing handwritten digits.

Once the model is trained, its performance is evaluated on a validation or test dataset, which consists of data the model has not seen before. This evaluation helps ensure that the model can generalize its learning to new inputs, rather than simply memorizing the training data. Metrics such as accuracy, precision, recall, and mean squared error are used to assess the quality of the model's predictions, depending on the task at hand.

Semi-supervised learning is a machine learning approach that combines elements of supervised and unsupervised learning. It leverages a small amount of labeled data alongside a larger set of unlabeled data to train a model. This method is particularly useful in situations where labeled data is scarce or expensive to obtain, but there is an abundance of unlabeled data.

In semi-supervised learning, the labeled data provides the model with explicit guidance about the relationships between inputs and outputs, similar to supervised learning. The unlabeled data, on the other hand, is used to capture the broader structure and patterns within the dataset, much like in unsupervised learning. By combining these two types of data, semi-supervised learning allows the model to generalize better and make accurate predictions, even with limited labeled data.

The process typically involves two steps. First, the model is trained using the labeled data, allowing it to learn the initial mappings between inputs and outputs. Next, the model is refined using the unlabeled data, which helps it uncover underlying patterns in the dataset. Techniques such as self-training, where the model uses its own predictions on unlabeled data as pseudo-labels for further training, are often employed. Graph-based methods and consistency regularization are also common strategies in semi-supervised learning, helping the model incorporate the unlabeled data more effectively.

Unsupervised learning is a type of machine learning where the model is trained on unlabeled data, meaning the data lacks predefined output labels or categories. The goal of unsupervised learning is to uncover hidden patterns, structures, or relationships in the data without explicit guidance. This is achieved by allowing the model to explore the data independently and group, cluster, or represent it in a meaningful way.

In unsupervised learning, the data consists only of inputs, and the model must learn to identify underlying structures based on similarities, differences, or statistical properties. For example, in a dataset containing customer purchase histories, the model might group customers with similar buying habits into distinct clusters, even though no explicit labels (e.g., "frequent shopper" or "occasional shopper") are provided. Accordingly, unsupervised learning is a powerful tool for discovering hidden structures and relationships in unlabeled data. It provides insights and data-driven groupings that inform decision-making or further analysis.

Self-supervised learning (SSL) is a machine learning approach that enables models to learn meaningful representations from unlabeled data by generating their own labels through pretext tasks. Unlike supervised learning, which relies on human-annotated datasets, SSL creates intrinsic learning objectives by leveraging patterns within the data itself. For example, in computer vision, an SSL model might learn by predicting the orientation of an image or reconstructing missing pixels, while in natural language processing (NLP), models like BERT learn by filling in masked words. Once trained, these models develop robust feature representations that can be fine-tuned on smaller labeled datasets for downstream tasks such as classification, segmentation, or anomaly detection. This approach significantly reduces the reliance on labeled data and improves model generalization.

SSL has proven highly effective in fields such as biomedical signal processing, where labeled data is often scarce. In cardiac electrophysiology, SSL can be applied to ECG or intracardiac signal analysis, where models learn to extract near-field signals without requiring manually labeled training data. By training on large datasets of raw unipolar signals, an SSL model can identify patterns distinguishing local myocardial activity (near-field) from distant noise (far-field), improving cardiac mapping accuracy. This method enhances scalability, making high-precision signal extraction feasible even in settings where multipolar signals or expert annotations are unavailable.

Ground truth refers to accurate and verified information used as a benchmark for evaluating the performance of models, systems, or algorithms. It represents the real-world, true output or label that a machine learning model seeks to predict or approximate. Ground truth is typically curated through reliable methods, such as manual annotation by experts, making it a trusted standard for comparison. In machine learning, ground truth is essential for supervised learning, where models are trained on labeled datasets. These labels provide the basis for calculating the error between a model's predictions and the actual outputs during training, enabling iterative improvements. For example, in an image classification task, ground truth consists of correct labels identifying the objects in each image, which are used to refine the model through metrics like accuracy or mean squared error.

Ground truth is also critical in evaluating model performance, as it allows for the assessment of accuracy, precision, recall, and other metrics against true outcomes. It plays a role in unsupervised learning as well, where it can be used post-training to validate the quality of results such as clustering. Examples of ground truth include annotated images in image recognition, transcribed text for speech-to-text systems, and precisely measured positions and trajectories in autonomous vehicle systems. However, obtaining accurate ground truth can be challenging, as it often requires significant resources, expertise, and manual effort. For instance, annotating medical images for diagnosing diseases may require skilled radiologists, making the process time-consuming and expensive. Even with expert input, inconsistencies or subjectivity in labeling can introduce errors into the ground truth, potentially affecting model training and evaluation.

Deep learning models are a subset of machine learning models that utilize artificial neural networks with multiple layers, or "deep" architectures, to learn and make predictions or decisions. These models are particularly well-suited for tasks involving large, complex datasets, such as image recognition, natural language processing, and speech recognition. The depth of a deep learning model refers to the number of layers in the neural network, with each layer learning progressively more abstract representations of the input data.

At the core of a deep learning model is the artificial neural network, which is inspired by the structure and functioning of the human brain. A neural network consists of layers of interconnected nodes, or "neurons." These include an input layer, where data is fed into the network; one or more hidden layers, which process the data; and an output layer, which generates the final predictions. Each neuron receives inputs, applies a mathematical operation using weights and biases, passes the result through an activation function, and sends the output to the next layer. The weights and biases are adjusted during training to minimize the error between the model's predictions and the true outcomes.

Deep learning models excel at feature extraction, as they can automatically identify important patterns and relationships in the data. For instance, in an image recognition task, the initial layers may detect simple features like edges and corners, while deeper layers recognize more complex patterns like shapes or objects. This hierarchical learning capability allows deep learning models to outperform traditional machine learning models on tasks involving unstructured data, such as images, audio, and text.

There are several types of deep learning models, each tailored to specific types of tasks. Convolutional Neural Networks (CNNs) are widely used for image-related tasks because they excel at processing spatial data and recognizing patterns in images. Recurrent Neural Networks (RNNs) and their variants, like Long Short-Term Memory (LSTM) networks, are designed for sequential data, making them suitable for tasks like time-series prediction and natural language processing. Transformer models, such as BERT and GPT, have revolutionized language-related tasks by enabling more efficient handling of long-range dependencies in text.

Training deep learning models requires large amounts of labeled data and significant computational power, as these models often involve millions or even billions of parameters. Optimization techniques like stochastic gradient descent and backpropagation are used to minimize the loss function, iteratively improving the model's performance. However, deep learning models can be prone to challenges such as overfitting, where the model performs well on training data but poorly on unseen data. Regularization techniques, dropout layers, and large-scale datasets are commonly used to address this.

Training a deep learning model to use multipolar signals for extracting near-field components of a unipolar signal involves leveraging the spatial richness of multipolar recordings to isolate localized electrical activity.

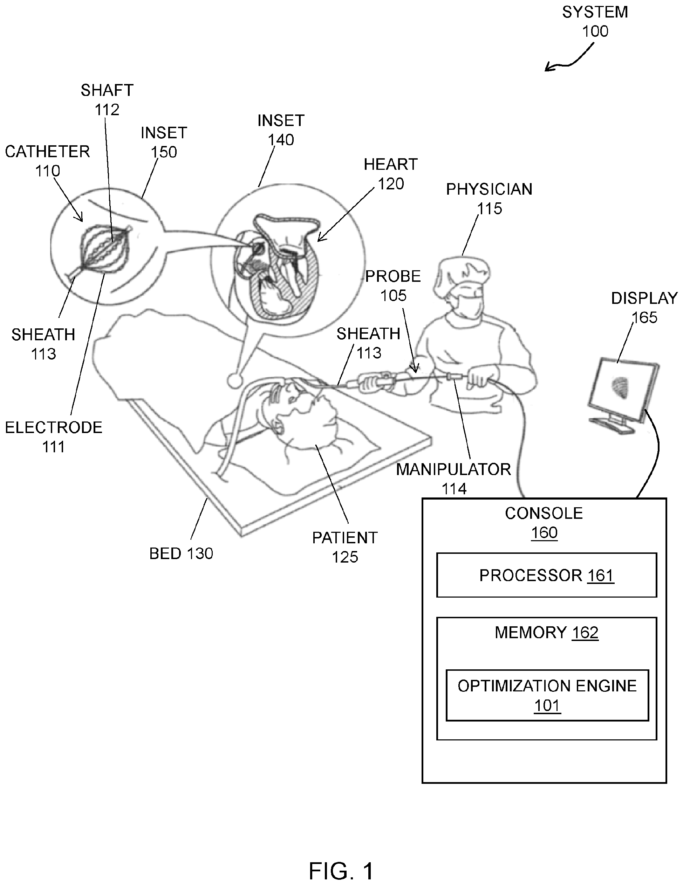

FIG. 1 is a diagram of an example system (e.g., medical device equipment), shown as a system 100, in which one or more features of the subject matter herein can be implemented according to one or more embodiments. All or part of the system 100 can be used to collect information (e.g., biometric data and/or a training dataset) and/or used to implement an optimization engine 101, which may be configured to implement any of the methods as described herein. For example, the optimization engine 101 may be configured to implement a two-stage deep learning approach for reducing far-field interference in unipolar cardiac signals. The optimization engine 101 can be defined as an optimization in which model parameters that best fit data and prior statistical knowledge are estimated in an iterative process.

The system 100, as illustrated, includes a probe 105 with a catheter 110 (including at least one electrode 111), a shaft 112, a sheath 113, and a manipulator 114. The system 100, as illustrated, also includes a physician 115 (or a medical professional or clinician), a heart 120, a patient 125, and a bed 130 (or a table). Note that insets 140 and 150 show the heart 120 and the catheter 110 in greater detail. The system 100 also, as illustrated, includes a console 160 (including one or more processors 161 and memories 162) and a display 165. Note further that each element and/or item of the system 100 is representative of one or more of that element and/or that item. The example of the system 100 shown in FIG. 1 can be modified to implement the embodiments disclosed herein. The disclosed embodiments can similarly be applied using other system components and settings. Additionally, the system 100 can include additional components, such as elements for sensing electrical activity, wired or wireless connectors, processing and display devices, or the like.

The system 100 can be utilized to detect, diagnose, and/or treat cardiac conditions, especially those relating to cardiac electrophysiology (e.g., using the optimization engine 101). Cardiac conditions, such as cardiac arrhythmias, persist as common and dangerous medical ailments, especially in the aging population. For instance, the system 100 can be part of a surgical system (e.g., CARTO^{®} system sold by Biosense Webster) that is configured to obtain biometric data (e.g., anatomical and electrical measurements of a patient's organ, such as the heart 120) and perform a cardiac ablation procedure. More particularly, treatments for cardiac conditions such as cardiac arrhythmia often require obtaining a detailed mapping of cardiac tissue, chambers, veins, arteries and/or electrical pathways. For example, a prerequisite for performing a catheter ablation (as described herein) successfully is that the cause of the cardiac arrhythmia is accurately located in a chamber of the heart 120. Such locating may be done via an electrophysiological investigation during which electrical potentials are detected spatially resolved with a mapping catheter (e.g., the catheter 110) introduced into the chamber of the heart 120. This electrophysiological investigation, the so-called electro-anatomical mapping, thus provides 3D mapping data which can be displayed on a monitor. In many cases, the mapping function and a treatment function (e.g., ablation) are provided by a single catheter or group of catheters such that the mapping catheter also operates as a treatment (e.g., ablation) catheter at the same time. In this case, the optimization engine 101 can be directly stored and executed by the catheter 110.

In patients (e.g., the patient 125) with normal sinus rhythm (NSR), the heart (e.g., the heart 120), which includes atrial, ventricular, and excitatory conduction tissue, is electrically excited to beat in a synchronous, patterned fashion. Note that this electrical excitement can be detected as intracardiac electrocardiogram (IC ECG) data or the like.

In patients (e.g., the patient 125) with a cardiac arrhythmia (e.g., atrial fibrillation or aFib), abnormal regions of cardiac tissue do not follow a synchronous beating cycle associated with normally conductive tissue, which is in contrast to patients with NSR. Instead, the abnormal regions of cardiac tissue aberrantly conduct to adjacent tissue, thereby disrupting the cardiac cycle into an asynchronous cardiac rhythm. Note that this asynchronous cardiac rhythm can also be detected as the IC ECG data. Such abnormal conduction has been previously known to occur at various regions of the heart 120, for example, in the region of the sino-atrial (SA) node, along the conduction pathways of the atrioventricular (AV) node, or in the cardiac muscle tissue forming the walls of the ventricular and atrial cardiac chambers. There are other conditions, such as flutter, where the pattern of abnormally conducting tissues lead to reentry paths such that the chamber beats in a regular pattern that can be multiple times the sinus rhythm.

In support of the system 100 detecting, diagnosing, and/or treating cardiac conditions, the probe 105 can be navigated by the physician 115 into the heart 120 of the patient 125 lying on the bed 130. For instance, the physician 115 can insert the shaft 112 through the sheath 113, while manipulating a distal end of the shaft 112 using the manipulator 114 near the proximal end of the catheter 110 and/or deflection from the sheath 113. As shown in an inset 140, the catheter 110 can be fitted at the distal end of the shaft 112. The catheter 110 can be inserted through the sheath 113 in a collapsed state and can be then expanded within the heart 120.

Generally, electrical activity at a point in the heart 120 may be typically measured by advancing the catheter 110 containing an electrical sensor at or near its distal tip (e.g., the at least one electrode 111) to that point in the heart 120, contacting the tissue with the sensor and acquiring data at that point. One drawback with mapping a cardiac chamber using a catheter type containing only a single, distal tip electrode is the long period of time required to accumulate data on a point-by-point basis over the requisite number of points required for a detailed map of the chamber as a whole. Accordingly, multiple-electrode catheters (e.g., the catheter 110) have been developed to simultaneously measure electrical activity at multiple points in the heart chamber.

The catheter 110, which can include the at least one electrode 111 and a catheter needle coupled onto a body thereof, can be configured to obtain biometric data, such as electrical signals of an intra-body organ (e.g., the heart 120), and/or to ablate tissue areas of thereof (e.g., a cardiac chamber of the heart 120). Note that the electrodes 111 are representative of any like elements, such as tracking coils, piezoelectric transducer, electrodes, or combination of elements configured to ablate the tissue areas or to obtain the biometric data. According to one or more embodiments, the catheter 110 can include one or more position sensors that used are to determine trajectory information. The trajectory information can be used to infer motion characteristics, such as the contractility of the tissue.

Biometric data (e.g., patient biometrics, patient data, or patient biometric data) can include one or more of local activation times (LATs), electrical activity, topology, bipolar mapping, reference activity, ventricle activity, dominant frequency, impedance, or the like. The LAT can be a point in time of a threshold activity corresponding to a local activation, calculated based on a normalized initial starting point. Electrical activity can be any applicable electrical signals that can be measured based on one or more thresholds and can be sensed and/or augmented based on signal to noise ratios and/or other filters. A topology can correspond to the physical structure of a body part or a portion of a body part and can correspond to changes in the physical structure relative to different parts of the body part or relative to different body parts. A dominant frequency can be a frequency or a range of frequency that is prevalent at a portion of a body part and can be different in different portions of the same body part. For example, the dominant frequency of a PV of a heart can be different than the dominant frequency of the right atrium of the same heart. Impedance can be the resistance measurement at a given area of a body part.

Examples of biometric data include, but are not limited to, patient identification data, IC ECG data, bipolar intracardiac reference signals, anatomical and electrical measurements, trajectory information, body surface (BS) ECG data, historical data, brain biometrics, blood pressure data, ultrasound signals, radio signals, audio signals, a two- or three-dimensional image data, blood glucose data, and temperature data. The biometrics data can be used, generally, to monitor, diagnosis, and treatment any number of various diseases, such as cardiovascular diseases (e.g., arrhythmias, cardiomyopathy, and coronary artery disease) and autoimmune diseases (e.g., type I and type II diabetes). Note that BS ECG data can include data and signals collected from electrodes on a surface of a patient, IC ECG data can include data and signals collected from electrodes within the patient, and ablation data can include data and signals collected from tissue that has been ablated. Further, BS ECG data, IC ECG data, and ablation data, along with catheter electrode position data, can be derived from one or more procedure recordings.

For example, the catheter 110 can use the electrodes 111 to implement intravascular ultrasound and/or MRI catheterization to image the heart 120 (e.g., obtain and process the biometric data). Inset 150 shows the catheter 110 in an enlarged view, inside a cardiac chamber of the heart 120. Although the catheter 110 is shown to be a point catheter, it will be understood that any shape that includes one or more electrodes 111 can be used to implement the exemplary embodiments disclosed herein.

Examples of the catheter 110 include, but are not limited to, a linear catheter with multiple electrodes, a balloon catheter including electrodes dispersed on multiple spines that shape the balloon, a lasso or loop catheter with multiple electrodes, a contact-force sensing catheter, or any other applicable shape or type. Linear catheters can be fully or partially elastic such that it can twist, bend, and or otherwise change its shape based on received signal and/or based on application of an external force (e.g., cardiac tissue) on the linear catheter. The balloon catheter can be designed such that when deployed into a patient's body, its electrodes can be held in intimate contact against an endocardial surface. As an example, a balloon catheter can be inserted into a lumen, such as a PV. The balloon catheter can be inserted into the PV in a deflated state, such that the balloon catheter does not occupy its maximum volume while being inserted into the PV. The balloon catheter can expand while inside the PV, such that those electrodes on the balloon catheter are in contact with an entire circular section of the PV. Such contact with an entire circular section of the PV, or any other lumen, can enable efficient imaging and/or ablation.

According to other examples, body patches and/or body surface electrodes may also be positioned on or proximate to a body of the patient 125. The catheter 110 with the one or more electrodes 111 can be positioned within the body (e.g., within the heart 120) and a position of the catheter 110 can be determined by the system 100 based on signals transmitted and received between the one or more electrodes 111 of the catheter 110 and the body patches and/or body surface electrodes. Additionally, the electrodes 111 can sense the biometric data from within the body of the patient 125, such as within the heart 120 (e.g., the electrodes 111 sense the electrical potential of the tissue in real time). The biometric data can be associated with the determined position of the catheter 110 such that a rendering of the patient's body part (e.g., the heart 120) can be displayed and show the biometric data overlaid on a shape of the body part.

The probe 105 and other items of the system 100 can be connected to the console 160. The console 160 can include any computing device, which employs the ML/AI algorithm (represented as the optimization engine 101). According to an exemplary embodiment, the console 160 includes the one or more processors 161 (any computing hardware) and the memory 162 (any non-transitory tangible media), where the one or more processors 161 execute computer instructions with respect the optimization engine 101 and the memory 162 stores these instructions for execution by the one or more processors 161. For instance, the console 160 can be configured to receive and/or store the biometric data on a database of the memory 162, process the biometric data, and determine if a given tissue area conducts electricity.

In some embodiments, the console 160 can be further programmed by the optimization engine 101 (in software) to carry out the functions of receiving inputs from previous ablation procedures, training a machine learning algorithm of the optimization engine to learn scenarios for the previous ablation procedures, and generating ablation indices for the scenarios.

According to one or more embodiment, the optimization engine 101 can be used to assess prior ablation procedures for effectiveness. In this regard, the optimization engine 101 cam be used outside of a present procedure.

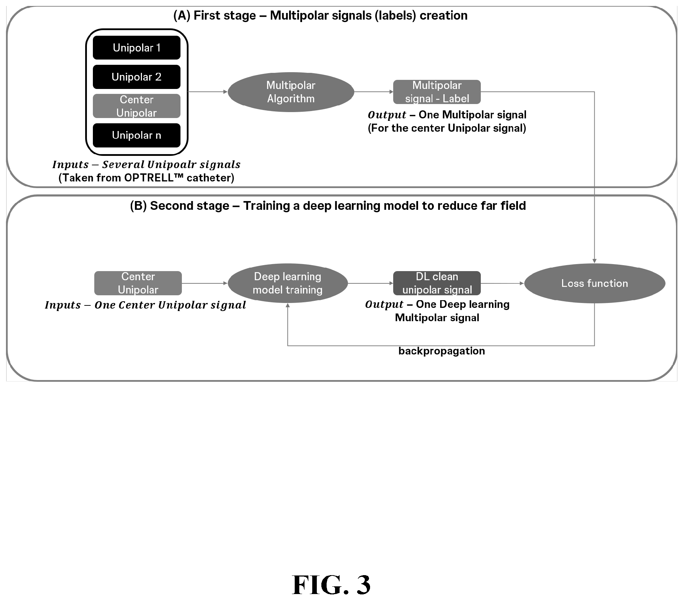

For instance, the optimization engine 101 can include the ML/AI algorithm (described herein with respect to FIGS. 3-7), that generates a multipolar signal that represents near-field activity with reduced interference. Once the multipolar signal is generated, the optimization engine 101 can process new unipolar signals in real time and remove far-field interference without requiring multiple unipolar inputs. Generally, the optimization engine 101 can provide one or more user interfaces, such as on behalf of the operating system or other application and/or directly as needed. The user interfaces include, but are not limited to, internet browsers, graphic user interfaces (GUls), window interfaces, and/or other visual interfaces for applications, operating systems, file folders, and the like. According to one or more embodiments, the optimization engine 101 can be external to the console 160 and can be located, for example, in the catheter 110, in an external device, in a mobile device, in a cloud-based device, or can be a standalone processor. In this regard, the optimization engine 101 can be transferable/downloaded in electronic form, over a network.

In an example, the console 160 can be any computing device, as noted herein, including software (e.g., the optimization engine 101) and/or hardware (e.g., the processor 161 and the memory 162), such as a general-purpose computer, with suitable front end and interface circuits for transmitting and receiving signals to and from the probe 105, as well as for controlling the other components of the system 100. For example, the front end and interface circuits include input/output (I/O) communication interfaces that enables the console 160 to receive signals from and/or transfer signals to the at least one electrode 111. The console 160 can include real-time noise reduction circuitry typically configured as a field programmable gate array (FPGA), followed by an analog-to-digital (A/D) ECG or electrocardiograph/electromyogram (EMG) signal conversion integrated circuit. The console 160 can pass the signal from an A/D ECG or EMG circuit to another processor and/or can be programmed to perform one or more functions disclosed herein.

The display 165, which can be any electronic device for the visual presentation of the biometric data, is connected to the console 160. According to an exemplary embodiment, during a procedure, the console 160 can facilitate on the display 165 a presentation of a body part rendering to the physician 115 and store data representing the body part rendering in the memory 162. For instance, maps depicting motion characteristics can be rendered/constructed based on the trajectory information sampled at a sufficient number of points in the heart 120. As an example, the display 165 can include a touchscreen that can be configured to accept inputs from the medical professional (e.g., physician 115), in addition to presenting the body part rendering.

In some embodiments, the physician 115 can manipulate the elements of the system 100 and/or the body part rendering using one or more input devices, such as a touch pad, a mouse, a keyboard, a gesture recognition apparatus, or the like. For example, an input device can be used to change a position of the catheter 110, such that rendering is updated. Note that the display 165 can be located at a same location or a remote location, such as a separate hospital or in separate healthcare provider networks.

According to one or more embodiments, the system 100 can also obtain the biometric data using ultrasound, computed tomography (CT), MRI, or other medical imaging techniques utilizing the catheter 110 or other medical equipment. For instance, the system 100 can obtain ECG data and/or anatomical and electrical measurements of the heart 120 (e.g., the biometric data) using one or more catheters 110 or other sensors. More particularly, the console 160 can be connected, by a cable, to BS electrodes, which include adhesive skin patches affixed to the patient 125. The BS electrodes can procure/generate the biometric data in the form of the BS ECG data. For instance, the processor 161 can determine position coordinates of the catheter 110 inside the body part (e.g., the heart 120) of the patient 125. The position coordinates may be based on impedances or electromagnetic fields measured between the body surface electrodes and the electrode 111 of the catheter 110 or other electromagnetic components. Additionally, or alternatively, location pads, which generate magnetic fields used for navigation, may be located on a surface of the bed 130 and may be separate from the bed 130. The biometric data can be transmitted to the console 160 and stored in the memory 162. Alternatively, or in addition, the biometric data may be transmitted to a server, which may be local or remote, using a network as further described herein.

According to one or more exemplary embodiments, the catheter 110 may be configured to ablate tissue areas of a cardiac chamber of the heart 120. Inset 150 shows the catheter 110 in an enlarged view, inside a cardiac chamber of the heart 120. For instance, ablation electrodes, such as the at least one electrode 111, may be configured to provide energy to tissue areas of an intra-body organ (e.g., the heart 120). The energy may be thermal energy and may cause damage to the tissue area starting from the surface of the tissue area and extending into the thickness of the tissue area. The biometric data with respect to ablation procedures (e.g., ablation tissues, ablation locations, etc.) can be considered ablation data.

According to an example, with respect to obtaining the biometric data, a multi-electrode catheter (e.g., the catheter 110) can be advanced into a chamber of the heart 120. Anteroposterior (AP) and lateral fluorograms can be obtained to establish the position and orientation of each of the electrodes. ECGs can be recorded from each of the electrodes 111 in contact with a cardiac surface relative to a temporal reference, such as the onset of the P-wave in sinus rhythm from a BS ECG and/or signals from electrodes 111 of the catheter 110 placed in the coronary sinus. The system, as further disclosed herein, may differentiate between those electrodes that register electrical activity and those that do not due to absence of close proximity to the endocardial wall. After initial ECGs are recorded, the catheter may be repositioned, and fluorograms and ECGs may be recorded again. An electrical map (e.g., via cardiac mapping) can then be constructed from iterations of the process above.

Cardiac mapping can be implemented using one or more techniques. Generally, mapping of cardiac areas such as cardiac regions, tissue, veins, arteries and/or electrical pathways of the heart 120 may result in identifying problem areas such as scar tissue, arrhythmia sources (e.g., electric rotors), healthy areas, and the like. Cardiac areas may be mapped such that a visual rendering of the mapped cardiac areas is provided using a display, as further disclosed herein. Additionally, cardiac mapping (which is an example of heart imaging) may include mapping based on one or more modalities such as, but not limited to LAT, local activation velocity, an electrical activity, a topology, a bipolar mapping, a dominant frequency, or an impedance. Data (e.g., biometric data) corresponding to multiple modalities may be captured using a catheter (e.g., the catheter 110) inserted into a patient's body and may be provided for rendering at the same time or at different times based on corresponding settings and/or preferences of the physician 115.

As an example of a first technique, cardiac mapping may be implemented by sensing an electrical property of heart tissue, for example, LAT, as a function of the precise location within the heart 120. The corresponding data (e.g., biometric data) may be acquired with one or more catheters (e.g., the catheter 110) that are advanced into the heart 120 and that have electrical and location sensors (e.g., the electrodes 111) in their distal tips. As specific examples, location and electrical activity may be initially measured on about 10 to about 20 points on the interior surface of the heart 120. These data points may be generally sufficient to generate a preliminary reconstruction or map of the cardiac surface to a satisfactory quality. The preliminary map may be combined with data taken at additional points to generate a more comprehensive map of the heart's electrical activity. In clinical settings, it is not uncommon to accumulate data at 100 or more sites (e.g., several thousand) to generate a detailed, comprehensive map of heart chamber electrical activity. The generated detailed map may then serve as the basis for deciding on a therapeutic course of action, for example, tissue ablation as described herein, to alter the propagation of the heart's electrical activity and to restore normal heart rhythm.

Further, cardiac mapping can be generated based on detection of intracardiac electrical potential fields (e.g., which is an example of IC ECG data and/or bipolar intracardiac reference signals). A non-contact technique to simultaneously acquire a large amount of cardiac electrical information may be implemented. For example, a catheter type having a distal end portion may be provided with a series of sensor electrodes distributed over its surface and connected to insulated electrical conductors for connection to signal sensing and processing means. The size and shape of the end portion may be such that the electrodes are spaced substantially away from the wall of the cardiac chamber. Intracardiac potential fields may be detected during a single cardiac beat. According to an example, the sensor electrodes may be distributed on a series of circumferences lying in planes spaced from each other. These planes may be perpendicular to the major axis of the end portion of the catheter. At least two additional electrodes may be provided adjacent at the ends of the major axis of the end portion. As a more specific example, the catheter may include four circumferences with eight electrodes spaced equiangularly on each circumference. Accordingly, in this specific implementation, the catheter may include at least 34 electrodes (32 circumferential and 2 end electrodes). As another more specific example, the catheter may include other multi-spline catheters, such as five soft flexible branches, eight radial splines, or a parallel splined pancake turner type (e.g., any of which may have a total of 42 electrodes).

As example of electrical or cardiac mapping, an electrophysiological cardiac mapping system and technique based on a non-contact and non-expanded multi-electrode catheter (e.g., the catheter 110) can be implemented. ECGs may be obtained with one or more catheters 110 having multiple electrodes (e.g., such as between 42 to 122 electrodes). According to this implementation, knowledge of the relative geometry of the probe and the endocardium can be obtained by an independent imaging modality, such as transesophageal echocardiography. After the independent imaging, non-contact electrodes may be used to measure cardiac surface potentials and construct maps therefrom (e.g., in some cases using bipolar intracardiac reference signals). This technique can include the following steps (after the independent imaging step): (a) measuring electrical potentials with a plurality of electrodes disposed on a probe positioned in the heart 120; (b) determining the geometric relationship of the probe surface and the endocardial surface and/or other reference; (c) generating a matrix of coefficients representing the geometric relationship of the probe surface and the endocardial surface; and (d) determining endocardial potentials based on the electrode potentials and the matrix of coefficients.

As another example of electrical or cardiac mapping, a technique and apparatus for mapping the electrical potential distribution of a heart chamber can be implemented. An intra-cardiac multi-electrode mapping catheter assembly can be inserted into the heart 120. The mapping catheter (e.g., the catheter 110) assembly can include a multi-electrode array with one or more integral reference electrodes (e.g., one or the electrodes 111) or a companion reference catheter.

According to one or more exemplary embodiments, the electrodes may be deployed in the form of a substantially spherical array, which may be spatially referenced to a point on the endocardial surface by the reference electrode or by the reference catheter this is brought into contact with the endocardial surface. The preferred electrode array catheter may carry a number of individual electrode sites (e.g., at least 24). Additionally, this example technique may be implemented with knowledge of the location of each of the electrode sites on the array, as well as knowledge of the cardiac geometry. These locations are preferably determined by a technique of impedance plethysmography.

In view of electrical or cardiac mapping and according to another example, the catheter 110 can be a heart mapping catheter assembly that may include an electrode array defining a number of electrode sites. The heart mapping catheter assembly can also include a lumen to accept a reference catheter having a distal tip electrode assembly that may be used to probe the heart wall. The map heart mapping catheter assembly can include a braid of insulated wires (e.g., having 24 to 64 wires in the braid), and each of the wires may be used to form electrode sites. The heart mapping catheter assembly may be readily positionable in the heart 120 to be used to acquire electrical activity information from a first set of non-contact electrode sites and/or a second set of in-contact electrode sites.

Further, according to another example, the catheter 110 that can implement mapping electrophysiological activity within the heart can include a distal tip that is adapted for delivery of a stimulating pulse for pacing the heart or an ablative electrode for ablating tissue in contact with the tip. This catheter 110 can further include at least one pair of orthogonal electrodes to generate a difference signal indicative of the local cardiac electrical activity adjacent the orthogonal electrodes.

As noted herein, the system 100 can be utilized to detect, diagnose, and/or treat cardiac conditions. In an example operation, a process for measuring electrophysiologic data in a heart chamber may be implemented by the system 100. The process may include, in part, positioning a set of active and passive electrodes into the heart 120, supplying current to the active electrodes, thereby generating an electric field in the heart chamber, and measuring the electric field at the passive electrode sites. The passive electrodes are contained in an array positioned on an inflatable balloon of a balloon catheter. In preferred embodiments, the array is said to have from 60 to 64 electrodes.

As another example operation, cardiac mapping may be implemented by the system 100 using one or more ultrasound transducers. The ultrasound transducers may be inserted into a patient's heart 120 and may collect a plurality of ultrasound slices (e.g., two dimensional or three-dimensional slices) at various locations and orientations within the heart 120. The location and orientation of a given ultrasound transducer may be known and the collected ultrasound slices may be stored such that they can be displayed at a later time. One or more ultrasound slices corresponding to the position of the probe 105 (e.g., a treatment catheter shown as catheter 110) at the later time may be displayed and the probe 105 may be overlaid onto the one or more ultrasound slices.

In view of the system 100, it is noted that cardiac arrhythmias, including atrial arrhythmias, may be of a multiwavelet reentrant type, characterized by multiple asynchronous loops of electrical impulses that are scattered about the atrial chamber and are often self-propagating (e.g., another example of the IC ECG data). Alternatively, or in addition to the multiwavelet reentrant type, cardiac arrhythmias may also have a focal origin, such as when an isolated region of tissue in an atrium fires autonomously in a rapid, repetitive fashion (e.g., another example of the IC ECG data). Ventricular tachycardia (V-tach or VT) is a tachycardia, or fast heart rhythm that originates in one of the ventricles of the heart. This is a potentially life-threatening arrhythmia because it may lead to ventricular fibrillation and sudden death.

For example, aFib occurs when the normal electrical impulses (e.g., another example of the IC ECG data) generated by the sinoatrial node are overwhelmed by disorganized electrical impulses (e.g., signal interference) that originate in the atria veins and PVs causing irregular impulses to be conducted to the ventricles. An irregular heartbeat results and may last from minutes to weeks, or even years. aFib is often a chronic condition that leads to a small increase in the risk of death often due to strokes. A line of treatment for aFib is medication that either slows the heart rate or revert the heart rhythm back to normal. Additionally, persons with aFib are often given anticoagulants to protect them from the risk of stroke. The use of such anticoagulants comes with its own risk of internal bleeding. In some patients, medication is not sufficient and their aFib is deemed to be drug-refractory, i.e., untreatable with standard pharmacological interventions. Synchronized electrical cardioversion may also be used to convert aFib to a normal heart rhythm. Alternatively, aFib patients are treated by catheter ablation.

A catheter ablation-based treatment may include mapping the electrical properties of heart tissue, especially the endocardium and the heart volume, and selectively ablating cardiac tissue by application of energy. Electrical or cardiac mapping (e.g., implemented by any electrophysiological cardiac mapping system and technique described herein) includes creating a map of electrical potentials (e.g., a voltage map) of the wave propagation along the heart tissue or a map of arrival times (e.g., a LAT map) to various tissue located points. Electrical or cardiac mapping (e.g., a cardiac map) may be used for detecting local heart tissue dysfunction. Ablations, such as those based on cardiac mapping, can cease or modify the propagation of unwanted electrical signals from one portion of the heart 120 to another.

The ablation process damages the unwanted electrical pathways by formation of non-conducting lesions. Various energy delivery modalities have been disclosed for forming lesions, and include use of microwave, laser and more commonly, radiofrequency energies to create conduction blocks along the cardiac tissue wall. Another example of an energy delivery technique includes irreversible electroporation (IRE), which provides high electric fields that damage cell membranes. In a two-step procedure (e.g., mapping followed by ablation) electrical activity at points within the heart 120 is typically sensed and measured by advancing the catheter 110 containing one or more electrical sensors (e.g., electrodes 111) into the heart 120 and obtaining/acquiring data at a multiplicity of points (e.g., as biometric data generally, or as ECG data specifically). This ECG data is then utilized to select the endocardial target areas, at which ablation is to be performed.

Cardiac ablation and other cardiac electrophysiological procedures have become increasingly complex as clinicians treat challenging conditions such as atrial fibrillation and ventricular tachycardia. The treatment of complex arrhythmias can now rely on the use of three-dimensional (3D) mapping systems to reconstruct the anatomy of the heart chamber of interest. In this regard, the optimization engine 101 employed by the system 100 herein manipulates and evaluates the biometric data generally, or the ECG data specifically, to produce improved tissue data that enables more accurate diagnosis, images, scans, and/or maps for treating an abnormal heartbeat or arrhythmia. For example, cardiologists rely upon software, such as the Complex Fractionated Atrial Electrograms (CFAE) module of the CARTO^{®} 3 3D mapping system, produced by Biosense Webster, Inc. (Diamond Bar, Calif.), to generate and analyze ECG data. The optimization engine 101 of the system 100 enhances this software to generate and analyze the improved biometric data, which further provide multiple pieces of information regarding electrophysiological properties of the heart 120 (including the scar tissue) that represent cardiac substrates (anatomical and functional) of aFib.

Accordingly, the system 100 can implement a 3D mapping system, such as CARTO^{®} 3 3D mapping system, to localize the potential arrhythmogenic substrate of the cardiomyopathy in terms of abnormal ECG detection. The substrate linked to these cardiac conditions is related to the presence of fragmented and prolonged ECGs in the endocardial and/or epicardial layers of the ventricular chambers (right and left). For instance, areas of low or medium voltage may exhibit ECG fragmentation and prolonged activities. Further, during sinus rhythm, areas of low or medium voltage may corresponds to a critical isthmus identified during sustained and organized ventricular arrhythmias (e.g., applies to non-tolerated ventricular tachycardias, as well as in the atria). In general, abnormal tissue is characterized by low-voltage ECGs. However, initial clinical experience in endo-epicardial mapping indicates that areas of low-voltage are not always present as the sole arrhythmogenic mechanism in such patients. In fact, areas of low or medium voltage may exhibit ECG fragmentation and prolonged activities during sinus rhythm, which corresponds to the critical isthmus identified during sustained and organized ventricular arrhythmias, e.g., applies only to non-tolerated ventricular tachycardias. Moreover, in many cases, ECG fragmentation and prolonged activities are observed in the regions showing a normal or near-normal voltage amplitude (>1-1.5 mV). Although the latter areas may be evaluated according to the voltage amplitude, they cannot be considered as normal according to the intracardiac signal, thus representing a true arrhythmogenic substrate. The 3D mapping may be able to localize the arrhythmogenic substrate on the endocardial and/or epicardial layer of the right/left ventricle, which may vary in distribution according to the extension of the main disease.

As another example operation, cardiac mapping may be implemented by the system 100 using one or more multiple-electrode catheters (e.g., the catheter 110). Multiple-electrode catheters are used to stimulate and map electrical activity in the heart 120 and to ablate sites of aberrant electrical activity. In use, the multiple-electrode catheter is inserted into a major vein or artery, e.g., femoral vein, and then guided into the chamber of the heart 120 of concern. A typical ablation procedure involves the insertion of the catheter 110 having at least one electrode 111 at its distal end, into a heart chamber. A reference electrode is provided, taped to the skin of the patient or by means of a second catheter that is positioned in or near the heart or selected from one or the other electrodes 111 of the catheter 110. Radio frequency (RF) current is applied to a tip electrode 111 of the ablating catheter 110, and current flows through the media that surrounds it (e.g., blood and tissue) toward the reference electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive. During this process, heating of the tip electrode 111 also occurs as a result of conduction from the heated tissue to the electrode itself. If the electrode temperature becomes sufficiently high, possibly above 60 degrees Celsius, a thin transparent coating of dehydrated blood protein can form on the surface of the electrode 111. If the temperature continues to rise, this dehydrated layer can become progressively thicker resulting in blood coagulation on the electrode surface. Because dehydrated biological material has a higher electrical resistance than endocardial tissue, impedance to the flow of electrical energy into the tissue also increases. If the impedance increases sufficiently, an impedance rise occurs, and the catheter 110 must be removed from the body and the tip electrode 111 cleaned.

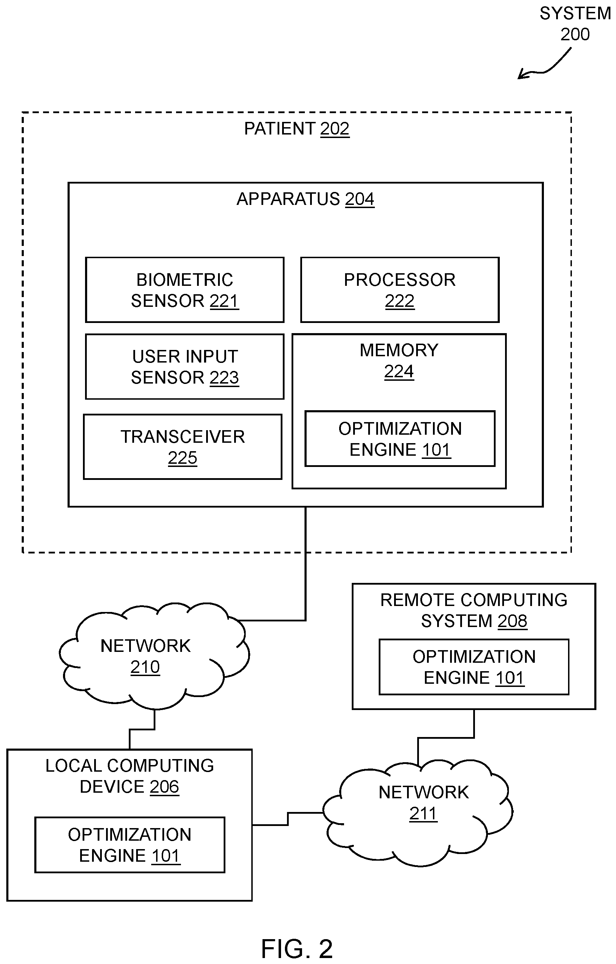

Turning now to FIG. 2, a diagram of a system 200 in which one or more features of the disclosure subject matter can be implemented is illustrated according to one or more exemplary embodiments. The system 200 includes, in relation to a patient 202 (e.g., an example of the patient 125 of FIG. 1), an apparatus 204, a local computing device 206, a remote computing system 208, a first network 210, and a second network 211. Further, the apparatus 204 can include a biometric sensor 221 (e.g., an example of the catheter 110 of FIG. 1), a processor 222, a user input (UI) sensor 223, a memory 224, and a transceiver 225. Note that the optimization engine 101 of FIG. 1 is reused in FIG. 2 for ease of explanation and brevity.

According to an embodiment, the apparatus 204 can be an example of the system 100 of FIG. 1, where the apparatus 204 can include both components that are internal to the patient and components that are external to the patient. According to another embodiment, the apparatus 204 can be an apparatus that is external to the patient 202 that includes an attachable patch (e.g., that attaches to a patient's skin). According to another embodiment, the apparatus 204 can be internal to a body of the patient 202 (e.g., subcutaneously implantable), where the apparatus 204 can be inserted into the patient 202 via any applicable manner including orally injecting, surgical insertion via a vein or artery, an endoscopic procedure, or a laparoscopic procedure. According to an embodiment, while a single apparatus 204 is shown in FIG. 2, example systems may include a plurality of apparatuses.

Accordingly, the apparatus 204, the local computing device 206, and/or the remote computing system 208 can be programed to execute computer instructions with respect the optimization engine 101. As an example, the memory 224 stores these instructions for execution by the processor 222 so that the apparatus 204 can receive and process the biometric data via the biometric sensor 201. In this way, the processor 222 and the memory 224 are representative of processors and memories of the local computing device 206 and/or the remote computing system 208.

The apparatus 204, local computing device 206, and/or the remote computing system 208 can be any combination of software and/or hardware that individually or collectively store, execute, and implement the optimization engine 101 and functions thereof. Further, the apparatus 204, local computing device 206, and/or the remote computing system 208 can be an electronic, computer framework comprising and/or employing any number and combination of computing device and networks utilizing various communication technologies, as described herein. The apparatus 204, local computing device 206, and/or the remote computing system 208 can be easily scalable, extensible, and modular, with the ability to change to different services or reconfigure some features independently of others.

The networks 210 and 211 can be a wired network, a wireless network, or include one or more wired and wireless networks. According to an embodiment, the network 210 is an example of a short-range network (e.g., local area network (LAN), or personal area network (PAN)). Information can be sent, via the network 210, between the apparatus 204 and the local computing device 206 using any one of various short-range wireless communication protocols, such as Bluetooth^{™}, Wi-Fi, Zigbee^{™}, Z-Wave^{™}, near field communications (NFC), ultra-band, or infrared (IR). Further, the network 211 is an example of one or more of an Intranet, a local area network (LAN), a wide area network (WAN), a metropolitan area network (MAN), a direct connection or series of connections, a cellular telephone network, or any other network or medium capable of facilitating communication between the local computing device 206 and the remote computing system 208. Information can be sent, via the network 211, using any one of various long-range wireless communication protocols (e.g., TCP/IP, HTTP, 3G, 4G/LTE, or 5G/New Radio). Note that for either network 210 and 211 wired connections can be implemented using Ethernet, Universal Serial Bus (USB), RJ-11 or any other wired connection and wireless connections can be implemented using Wi-Fi, WiMAX, and Bluetooth^{™}, infrared, cellular networks, satellite or any other wireless connection methodology.

In operation, the apparatus 204 can continually or periodically obtain, monitor, store, process, and communicate, via network 210, the biometric data associated with the patient 202. Further, the apparatus 204, local computing device 206, and/or the remote computing system 208 are in communication through the networks 210 and 211 (e.g., the local computing device 206 can be configured as a gateway between the apparatus 204 and the remote computing system 208). For instance, the apparatus 204 can be an example of the system 100 of FIG. 1 configured to communicate with the local computing device 206 via the network 210. The local computing device 206 can be, for example, a stationary/standalone device, a base station, a desktop/laptop computer, a smart phone, a smartwatch, a tablet, or other device configured to communicate with other devices via networks 210 and 211. The remote computing system 208, implemented as a physical server on or connected to the network 211 or as a virtual server in a public cloud computing provider (e.g., Amazon Web Services (AWS) ^{®}) of the network 211, can be configured to communicate with the local computing device 206 via the network 211. Thus, the biometric data associated with the patient 202 can be communicated throughout the system 200.

Elements of the apparatus 204 are now described. The biometric sensor 221 may include, for example, one or more transducers configured to convert one or more environmental conditions into an electrical signal, such that different types of biometric data are observed/obtained/acquired. For example, the biometric sensor 221 can include one or more of an electrode (e.g., the electrode 111 of FIG. 1), a temperature sensor (e.g., thermocouple), a blood pressure sensor, a blood glucose sensor, a blood oxygen sensor, a pH sensor, an accelerometer, and a microphone.

The processor 222, in executing the optimization engine 101, can be configured to receive, process, and manage the biometric data acquired by the biometric sensor 221, and communicate the biometric data to the memory 224 for storage (e.g., on a database therein) and/or across the network 210 via the transceiver 225 (e.g., to a database thereof). Biometric data from one or more other apparatuses 204 can also be received by the processor 222 through the transceiver 225. Also, as described in more detail herein, the processor 222 may be configured to respond selectively to different tapping patterns (e.g., a single tap or a double tap) received from the UI sensor 223, such that different tasks of a patch (e.g., acquisition, storing, or transmission of data) can be activated based on the detected pattern. In some embodiments, the processor 222 can generate audible feedback with respect to detecting a gesture.

The UI sensor 223 includes, for example, a piezoelectric sensor or a capacitive sensor configured to receive a user input, such as a tapping or touching. For example, the UI sensor 223 can be controlled to implement a capacitive coupling, in response to tapping or touching a surface of the apparatus 204 by the patient 202. Gesture recognition may be implemented via any one of various capacitive types, such as resistive capacitive, surface capacitive, projected capacitive, surface acoustic wave, piezoelectric and infra-red touching. Capacitive sensors may be disposed at a small area or over a length of the surface, such that the tapping or touching of the surface activates the monitoring device.

The memory 224 is any non-transitory tangible media, such as magnetic, optical, or electronic memory (e.g., any suitable volatile and/or non-volatile memory, such as random-access memory or a hard disk drive). The memory 224 stores the computer instructions for execution by the processor 222.

The transceiver 225 may include a separate transmitter and a separate receiver. Alternatively, the transceiver 225 may include a transmitter and receiver integrated into a single device.

In operation, the apparatus 204, utilizing the optimization engine 101, observes/obtains the biometric data of the patient 202 via the biometric sensor 221, stores the biometric data in the memory, and shares this biometric data across the system 200 via the transceiver 225. The optimization engine 101 can then utilize models (e.g., the parametric model), algorithms (e.g., the unsupervised and/or supervised ML/AI algorithm), neural networks, machine learning, and/or artificial intelligence to generate and provide a multipolar signal that represents near-field activity with reduced interference to allow for processing new unipolar signals in real time and remove far-field interference without requiring multiple unipolar inputs, to enable single-signal far-field suppression, reduce processing loads for the system 100, and transform operations of the system 100 to provide more accurate mapping and enhanced arrhythmia detection.

In an embodiment, a two-stage approach is utilized. In this approach a first model is used to generate high-quality near-field components from multipolar signals, which then serve as ground truth for training a second model that operates solely on unipolar signals.

For example, FIG. 3 illustrates a block diagram depicting the two-stage methodology for reducing far-field interference in unipolar cardiac signals using a deep learning-based approach. This figure outlines the process of first generating multipolar signals using an existing Multipolar algorithm (Stage A) and then utilizing these multipolar signals as training labels for a deep learning model that learns to extract near-field components from unipolar signals (Stage B).

In the first stage (Stage A), the process begins with the acquisition of multiple unipolar signals from a cardiac mapping catheter. These unipolar signals contain both near-field components (local myocardial activity) and far-field components (electrical activity originating from distant cardiac regions or extracardiac sources). To create training labels for deep learning, an existing Multipolar algorithm is applied to these unipolar signals. This algorithm estimates and removes far-field interference by leveraging multiple surrounding unipolar signals, ultimately producing a multipolar signal that retains only near-field components while significantly reducing the influence of far-field sources. These multipolar signals are referred to as unipolar signals without far-field interference and serve as high-quality reference signals for training the deep learning model.

In the second stage (Stage B), the deep learning model is trained to reduce far-field interference from unipolar signals by learning from the multipolar signals generated in Stage A. The training dataset consists of raw unipolar signals as inputs and the corresponding multipolar signals as ground truth labels. The deep learning model is optimized to predict a near-field-enhanced signal from a single unipolar input, mimicking the output of the multipolar algorithm without requiring multiple unipolar signals during inference. The architecture of the deep learning model may include Convolutional Neural Networks (CNNs) to extract spatial features, Recurrent Neural Networks (RNNs) such as Long Short-Term Memory (LSTM) networks to capture temporal dependencies, and attention mechanisms to enhance feature selection and improve accuracy. The training process involves supervised learning, where a loss function (such as Mean Squared Error (MSE) or a custom signal reconstruction loss) is minimized to ensure that the model accurately removes far-field interference while preserving near-field signal integrity.

Once the deep learning model is trained, it can be deployed in real-time electrophysiological applications to process single unipolar signals and generate far-field-reduced outputs, eliminating the need for multipolar signal acquisition during real-world use. The advantage of this approach lies in its ability to generalize across different catheter types and cardiac conditions, making it a scalable and adaptable solution for enhancing cardiac signal clarity.

FIG. 3 visually represents the structured pipeline of this two-stage process, illustrating how traditional multipolar signal processing techniques are first used to generate high-quality training data, which is then leveraged to train a deep learning model capable of far-field suppression without requiring additional unipolar signals at runtime. This method enables enhanced cardiac mapping accuracy, improves the interpretability of electrophysiological recordings, and provides a clinically viable solution for real-time signal analysis in arrhythmia diagnosis and catheter ablation procedures.

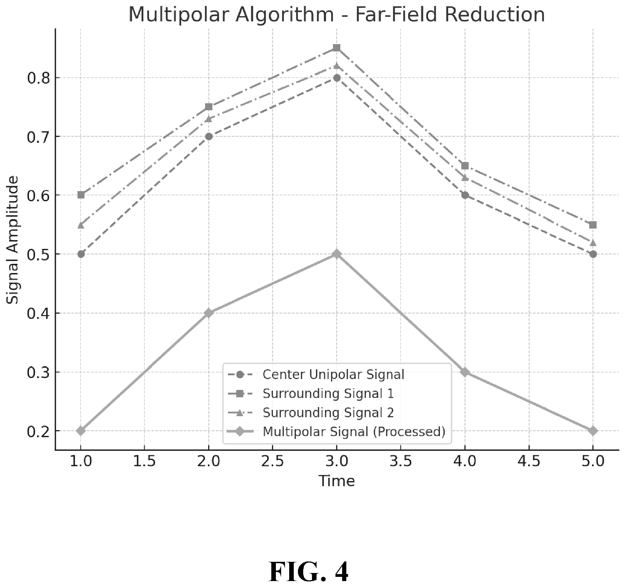

FIG. 4 is a diagram illustrating the Multipolar Algorithm in action. It shows how the center unipolar signal (dashed line) and surrounding unipolar signals (dash-dot lines) contain far-field interference. The algorithm estimates and subtracts the far-field component using the surrounding signals, resulting in the Multipolar Signal (Processed), which has reduced far-field interference.

The Multipolar algorithm is a signal-processing technique used in electrophysiology to remove far-field interference from unipolar signals. Far-field interference occurs when a unipolar electrode picks up electrical activity from distant cardiac sources, which can obscure the local electrical signals needed for precise mapping and diagnosis. To address this issue, the Multipolar algorithm processes multiple unipolar signals recorded from different electrodes, including a center unipolar signal, which is the primary signal of interest, and several surrounding unipolar signals from nearby electrodes. The algorithm assumes that the far-field component is present in all these signals, though with variations in amplitude and phase depending on the electrode's spatial location. By analyzing these signals collectively, the algorithm estimates the far-field interference and subtracts it from the center unipolar signal, producing a Multipolar signal, which represents the unipolar signal with reduced far-field interference.

Mathematically, the Multipolar algorithm can be described as subtracting an estimated far-field component from the center unipolar signal. This estimation is derived from surrounding unipolar signals using linear or statistical models that capture the correlations between the signals. Unlike deep learning approaches, which learn complex patterns from data, the Multipolar algorithm relies on predefined mathematical computations to model the far-field component. While effective, this method has several limitations. First, it requires multiple unipolar signals as input, making it inapplicable in cases where only a single unipolar signal is available. Additionally, it is catheter-dependent, as it is specifically designed for use with catheters such as the OPTRELL^{™} catheter, limiting its applicability to other catheter designs with different electrode configurations. Furthermore, its performance degrades when processing unipolar signals recorded during atrial fibrillation (AF) because the variability in far-field signals makes it more difficult to estimate and remove interference accurately. Another limitation is its sensitivity to electrode placement; for example, electrodes positioned at the catheter's circumference may not provide optimal far-field subtraction, reducing the algorithm's effectiveness.

The deep learning-based approach overcomes these limitations by eliminating the need for multiple unipolar signals. Instead of relying on multiple electrodes to estimate and remove the far-field component, the deep learning model is trained to recognize far-field interference within a single unipolar signal and remove it directly. This allows for more flexible and generalizable signal processing that can be applied across different catheter systems and electrophysiological conditions, ultimately improving the accuracy and usability of far-field reduction in clinical and research settings.

The first stage involves generating training data by leveraging the Multipolar algorithm, which produces multipolar signals-unipolar signals without far-field interference. This is achieved by acquiring multiple unipolar signals, including a center unipolar signal and adjacent signals, and applying the algorithm to estimate and subtract the far-field component. The resulting multipolar signals serve as ground truth labels for training the deep learning model.

In the second stage, the deep learning model is trained using these multipolar signals as labels. The model receives a single unipolar signal as input and is optimized to produce an output that approximates the corresponding multipolar signal, effectively removing far-field interference. The training process employs supervised learning, where the model minimizes the difference between its output and the multipolar signal labels. The deep learning model architecture may be implemented using convolutional neural networks (CNNs), recurrent neural networks (RNNs), transformers, or hybrid approaches designed to capture temporal and spatial dependencies within unipolar signals. Once trained, the model can process real-time unipolar signals and generate far-field-reduced signals without requiring multiple input signals.

The architecture of the second-stage model is designed to focus on the temporal characteristics of unipolar signals, extracting features indicative of near-field activity. Recurrent neural networks (RNNs), such as Long Short-Term Memory (LSTM) networks or Gated Recurrent Units (GRUs), may be employed to model the sequential nature of the signals, capturing dynamic patterns like activation timing and wave propagation. Fully connected layers integrate these temporal features, identifying patterns relevant to near-field signal extraction, while dropout layers are often included to prevent overfitting and improve generalization. In some cases, attention mechanisms are used to focus on the most significant portions of the signal, dynamically weighting temporal regions that are likely to contain near-field components. The input unipolar signals are preprocessed into sequences or tensors, ensuring they are compatible with the model architecture.

The training process for the second-stage model uses the ground truth generated by the first-stage model as supervision. Unipolar signals are paired with their corresponding multipolar signals, as generated by the first stage, and serve as the labeled training dataset. These unipolar signals may be preprocessed to remove noise, normalize amplitudes, and segment them into consistent time windows, ensuring data uniformity. Other preprocessing may be applied.

For example, since this stage lacks the spatial context provided by multipolar signals, preprocessing focuses on temporal alignment, noise suppression, and feature enhancement to isolate near-field activity while minimizing far-field contributions. One critical step is signal synchronization and alignment, which ensures that unipolar signals are referenced to a common time scale, correcting for latency variations across recordings. Additionally, noise filtering and artifact removal techniques, such as bandpass filtering, wavelet decomposition, and adaptive filtering, help eliminate unwanted low-frequency baseline drift, power line interference, and muscle artifacts, ensuring cleaner signal input.

In some instances, to maintain consistency, amplitude normalization and scaling techniques, including z-score normalization and baseline drift correction, standardize signal amplitudes and remove slow fluctuations that could distort near-field signals. Further, feature enhancement methods such as differential signal computation, spectral analysis (Fourier or Wavelet Transform), and energy-based filtering may help emphasize localized near-field activity by isolating higher-frequency components and suppressing diffuse far-field contributions. Segmentation and time-windowing play a crucial role by structuring the data into meaningful time frames, either through fixed-length segments or adaptive windowing techniques that account for heart rate variability. To improve robustness and generalization, data augmentation strategies like synthetic noise addition, time shifting, and amplitude scaling introduce variations that may help the model learn more effectively. These preprocessing steps may ensure that the second-stage model receives clean, well-structured unipolar signals, enabling it to accurately extract near-field components without requiring multipolar data.

The model is trained to minimize a loss function, such as mean squared error or mean absolute error, which quantifies the difference between the model's predicted outputs and the pseudo ground truth. Backpropagation and gradient descent are used to optimize the model's parameters iteratively, refining its ability to predict near-field components. To improve robustness, data augmentation techniques such as adding synthetic noise, shifting signal timing, or scaling amplitudes may be applied, enabling the model to handle variability in real-world data.

After training, the second-stage model is evaluated on unseen data to ensure its effectiveness. Metrics such as signal reconstruction accuracy and localization accuracy may be used to validate its performance, while robustness tests ensure that it can handle noisy or variable input signals. The outputs of the second-stage model are also compared with the Multipolar signals generated by the first-stage model to confirm consistency and reliability. This evaluation process ensures that the second-stage model can deliver accurate near-field predictions independently of multipolar inputs.

This second stage offers significant advantages by allowing near-field signal extraction from unipolar signals. Despite relying on less spatially detailed inputs, the second-stage model achieves high accuracy by learning from the ground truth generated by the first stage. This independence from multipolar signals makes the method scalable and suitable for real-world applications, especially in environments where multipolar recording systems are unavailable. For example, when using a non-supporting Multipolar catheter or is scenarios when the Multipolar algorithm has limitations, the second-stage model inherits the precision and reliability of the first model while operating on more accessible data, making it an efficient and robust solution for near-field signal extraction.

In practical terms, this approach allows the second model to generalize well to real-world scenarios where only unipolar signals are available. It scales effectively, operates efficiently, and enables near-field signal extraction in applications such as cardiac mapping or neural signal processing. The synergy between the two models-where the first model creates a reliable ground truth and the second model learns to replicate this capability with unipolar inputs-ensures robust, scalable, and high-accuracy near-field signal extraction, combining the strengths of both multipolar data and deep learning techniques.

In addition, this two-stage approach improves upon traditional techniques in several key ways. First, it leverages the spatial richness of multipolar signals to generate high-quality near-field components, which traditional unipolar-based methods lack. Second, the reliance on deep learning eliminates the need for manual feature engineering, automating the extraction process and allowing the models to learn complex spatial-temporal patterns directly from the data. Third, the staged design ensures scalability by enabling the second model to operate independently of multipolar inputs, making the method practical for real-world scenarios where multipolar signals may be unavailable or have other limitations.

By combining the strengths of multipolar data, deep learning, and staged training, this approach achieves a level of precision and robustness that conventional methods cannot match. The creation of unipolar signals without far field, using deep learning model where the labels are Multipolar signals created using the Multipolar algorithm ensures accurate, scalable, and real-time near-field signal extraction, suitable for applications such as cardiac mapping, neural signal processing, and other fields requiring precise localization of electrical activity. This innovation fundamentally transforms the extraction process by making it more accessible, accurate, and adaptable to diverse use cases.

Accordingly, this method surpasses conventional approaches by combining the spatial richness of multipolar signals, advanced feature extraction, and the analytical power of deep learning. It provides enhanced precision, robustness, and adaptability, making it a transformative tool for extracting near-field components in biomedical signal processing.

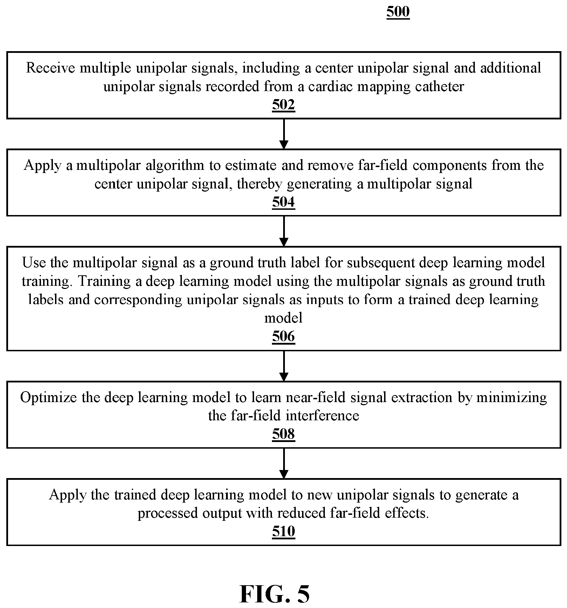

FIG. 5 illustrates a flowchart depicting the two-stage method for reducing far-field interference in unipolar cardiac signals using a deep learning-based approach. This process 500 involves receiving unipolar signals, applying a multipolar algorithm to generate training labels, training a deep learning model, and deploying the trained model for real-time near-field signal extraction.

In the first stage, at step 502, multiple unipolar signals are received from a cardiac mapping catheter, including a center unipolar signal and additional surrounding unipolar signals. The center unipolar signal represents the primary recorded electrical activity, while the surrounding unipolar signals provide spatial context necessary for far-field estimation. The signals may be acquired from various catheter configurations, such as circular, linear, or grid-like electrode arrays. At step 504, a multipolar algorithm is applied to estimate and remove far-field components from the center unipolar signal. The algorithm calculates a far-field estimate based on the surrounding unipolar signals, subtracts the estimated far-field component from the center unipolar signal, and generates a multipolar signal, which represents a far-field-reduced version of the original unipolar signal. This multipolar signal serves as the ground truth label for deep learning model training. The use of multipolar signals enhances the model's ability to generalize far-field suppression across different recording conditions, catheter designs, and arrhythmic states such as atrial fibrillation or ventricular tachycardia.

In the second stage, at step 506, the multipolar signal is used as a ground truth label for training a deep learning model. The model is trained using a dataset of unipolar signals as inputs and corresponding multipolar signals as labels to learn how to extract near-field components while minimizing far-field interference. Prior to training, the raw unipolar signals undergo preprocessing to enhance model performance. Signal synchronization ensures that input unipolar signals are aligned with their corresponding multipolar signals. Noise filtering techniques, including bandpass filtering, wavelet decomposition, and adaptive filtering, remove baseline drift, muscle artifacts, and external interference. Amplitude normalization techniques, such as z-score normalization or min-max scaling, standardize signal intensities across different recordings. Segmentation and time-windowing structure the training data into fixed-length time frames, accounting for heart rate variability. Data augmentation methods, such as synthetic noise injection, time shifting, and amplitude scaling, improve the model's ability to generalize across varying clinical conditions.

At step 508, the deep learning model undergoes optimization to enhance near-field signal extraction. The model minimizes a loss function, such as Mean Squared Error (MSE), Mean Absolute Error (MAE), or a custom signal reconstruction loss, to refine its ability to suppress far-field interference while preserving near-field components. Optimization techniques, including gradient descent and backpropagation, dynamically adjust network weights, while regularization methods such as dropout layers and batch normalization prevent overfitting. The model architecture may consist of convolutional neural networks (CNNs) for learning spatial features of unipolar signals, recurrent neural networks (RNNs) such as Long Short-Term Memory (LSTM) units or Gated Recurrent Units (GRUs) for capturing temporal dependencies, and transformer-based architectures that incorporate attention mechanisms to improve focus on relevant portions of the signal. Hybrid models combining CNNs and RNNs may also be employed to effectively capture both spatial and temporal features.

At step 510, the trained deep learning model is deployed for real-time far-field suppression in new unipolar signals. The trained model processes a single unipolar signal as input and generates a processed output, where far-field effects have been reduced, thereby providing a near-field-enhanced cardiac signal. At step 510, the processed signal can be integrated into 3D electroanatomical mapping systems for visualization, used for real-time arrhythmia detection during catheter ablation procedures, displayed on electrophysiology recording systems to assist in diagnosing conduction abnormalities, or stored for post-procedural analysis to assess signal quality improvements.

This two-stage model eliminates the need for multiple unipolar signals by training a model capable of far-field suppression using only a single unipolar input. The method generalizes across different catheter types, overcoming the limitations of catheter-dependent multipolar algorithms. Additionally, it improves accuracy in complex arrhythmic cases such as atrial fibrillation, where traditional multipolar methods perform poorly. The real-time applicability of this approach enables seamless integration into clinical cardiac mapping systems, providing electrophysiologists with more accurate and reliable data for arrhythmia diagnosis and treatment.

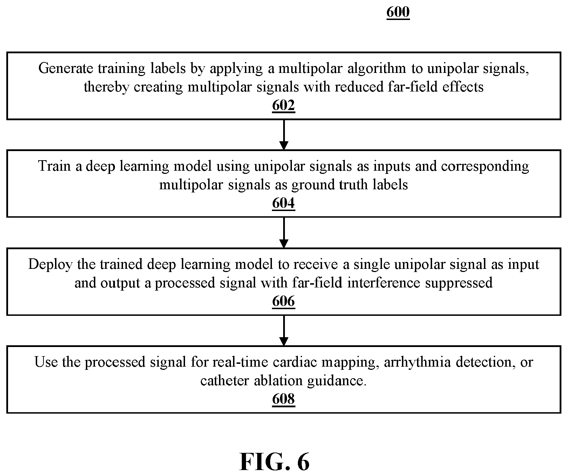

FIG. 6 illustrates a flowchart describing the overall workflow of training and deploying a deep learning model for reducing far-field interference in unipolar cardiac signals. This process 600 involves generating training labels using a multipolar algorithm, training a deep learning model with these labels, deploying the trained model for real-time far-field suppression, and using the processed signal for electrophysiological applications, such as cardiac mapping, arrhythmia detection, and catheter ablation guidance.

At step 602, training labels are generated by applying a multipolar algorithm to unipolar signals, thereby creating multipolar signals with reduced far-field effects. The multipolar algorithm operates by using multiple unipolar signals recorded from an electrophysiology catheter to estimate and subtract far-field interference, resulting in a near-field-enhanced multipolar signal. These multipolar signals serve as high-quality training labels for the subsequent deep learning model training phase. By leveraging multipolar signals as ground truth, the deep learning model can learn to extract near-field components effectively, even when only a single unipolar signal is available.

At step 604, a deep learning model is trained using unipolar signals as inputs and the previously generated multipolar signals as ground truth labels. The training process involves optimizing the model to predict the corresponding near-field-enhanced signal when given a raw unipolar input. To improve accuracy, the model undergoes various preprocessing steps, including noise reduction, amplitude normalization, and segmentation. The deep learning model architecture may include convolutional neural networks (CNNs) to extract spatial features, recurrent neural networks (RNNs) such as Long Short-Term Memory (LSTM) networks to capture temporal dependencies, or attention-based transformer models to enhance feature learning. The model is trained using loss functions that minimize the difference between its predicted output and the ground truth multipolar signal, ensuring effective far-field suppression.

At step 606, the trained deep learning model is deployed for real-time far-field suppression. In deployment, the model receives a single unipolar signal as input and outputs a processed signal where far-field interference has been minimized, effectively enhancing near-field clarity. Unlike traditional multipolar algorithms that require multiple unipolar inputs, the deep learning model is capable of extracting near-field components from a single unipolar input, making it more adaptable across different catheter designs and electrophysiological conditions.

At step 608, the processed signal is used for real-time cardiac applications, including electroanatomical mapping, arrhythmia detection, and catheter ablation guidance. By integrating the processed signal into a 3D cardiac mapping system, clinicians can visualize localized electrical activity with improved accuracy, aiding in the diagnosis and treatment of arrhythmias such as atrial fibrillation or ventricular tachycardia. During catheter ablation procedures, the model assists in pinpointing arrhythmic sites, enabling more precise ablation while preserving healthy myocardial tissue. Additionally, the far-field-reduced signal can be stored for post-procedural analysis, allowing for further evaluation of conduction abnormalities and procedural outcomes.

FIG. 6 provides a structured representation of the full pipeline, from generating training labels using multipolar signals to deploying a deep learning model for real-time electrophysiological applications. This approach overcomes the limitations of traditional far-field suppression techniques by eliminating the need for multiple unipolar signals, allowing for more flexible and scalable signal processing across different cardiac conditions and catheter types. The integration of deep learning ensures robust near-field signal extraction, improving the precision and efficiency of modern cardiac mapping and ablation procedures.

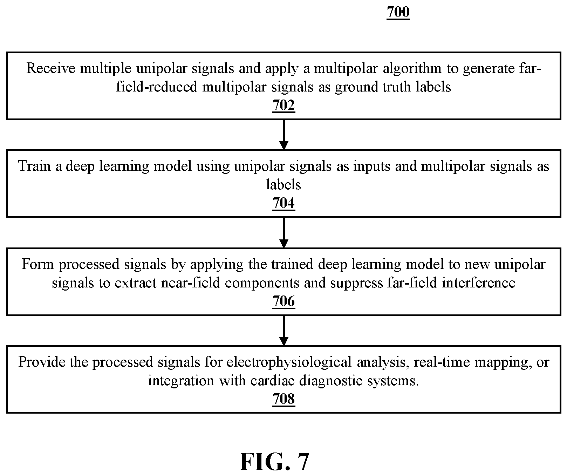

FIG. 7 illustrates a flowchart outlining the method for training and deploying a deep learning model to suppress far-field interference in unipolar cardiac signals. The process 700 begins with acquiring unipolar signals and generating training labels using a multipolar algorithm, followed by training a deep learning model, applying the trained model to new unipolar signals, and integrating the processed signals into electrophysiological analysis and cardiac diagnostic systems.

At step 702, multiple unipolar signals are received and processed using a multipolar algorithm to generate far-field-reduced multipolar signals as ground truth labels. The multipolar algorithm estimates far-field interference by analyzing multiple spatially distributed unipolar signals recorded from a cardiac mapping catheter. The far-field estimate is then subtracted from the center unipolar signal, resulting in a multipolar signal with enhanced near-field clarity. These multipolar signals serve as training labels for the deep learning model, allowing it to learn how to isolate near-field components from raw unipolar signals.

At step 704, a deep learning model is trained using unipolar signals as inputs and the previously generated multipolar signals as ground truth labels. The model learns to extract near-field components by minimizing far-field interference through supervised learning. During training, preprocessing techniques such as bandpass filtering, wavelet transformation, and amplitude normalization are applied to enhance signal quality. The model architecture may include convolutional neural networks (CNNs) to capture spatial features, recurrent neural networks (RNNs) such as Long Short-Term Memory (LSTM) networks to analyze temporal dependencies, and transformer-based attention mechanisms to improve feature extraction. The model is optimized using loss functions such as Mean Squared Error (MSE) or custom signal reconstruction losses that prioritize near-field signal preservation.

At step 706, the trained deep learning model is deployed to process new unipolar signals by extracting near-field components while suppressing far-field interference. Unlike traditional multipolar algorithms that require multiple unipolar inputs for processing, the trained deep learning model operates on a single unipolar signal, making it more versatile and adaptable across different catheter types and patient conditions. The model ensures that the processed signal retains its physiological integrity while minimizing distortions caused by far-field interference.

At step 708, the processed signals are provided for electrophysiological analysis, real-time mapping, or integration with cardiac diagnostic systems. In clinical applications, the processed signals can be incorporated into electroanatomical mapping platforms to improve arrhythmia diagnosis, assist in catheter ablation procedures, and enhance the precision of electrophysiological studies. By reducing far-field interference, the model allows electrophysiologists to more accurately visualize localized cardiac activity, improving the reliability of electrical activation maps and voltage maps. Additionally, the processed signals can be stored for post-procedural analysis, enabling retrospective evaluation of conduction abnormalities and treatment effectiveness.

FIG. 7 provides a structured representation of the entire deep learning-based workflow, demonstrating how unipolar signals are transformed through a multipolar algorithm into training labels, how a deep learning model is trained to suppress far-field interference, and how the trained model is deployed for real-time electrophysiological applications. This approach enhances cardiac signal processing by eliminating the need for multipolar recordings during real-time use, improving adaptability across different mapping systems, and offering a scalable solution for automated near-field signal extraction.

In certain embodiments, the deep learning model is configured to support transfer learning to facilitate adaptation to new patient data with minimal retraining. Transfer learning refers to a machine learning technique where a model developed for a task is reused as the starting point for a model on a second task, enabling faster and more efficient model adaptation. In the context of the present invention, the deep learning model initially trained using multipolar-derived ground truth signals from a first population of patients can be fine-tuned using a relatively small amount of additional unipolar signal data collected from a new patient or patient cohort.

During deployment, the pretrained model weights capture generalized features relevant to distinguishing near-field and far-field components in cardiac unipolar signals. To adapt the model to a new patient's specific cardiac signal characteristics (which may differ due to anatomical variations, disease state, electrode placement, or other factors), the system may perform transfer learning by selectively updating only a subset of the model parameters, such as the weights of later layers in the neural network, while freezing the earlier layers. This allows the model to retain its general feature extraction capabilities while refining its output to better match the new patient's signal morphology.

In some embodiments, transfer learning may involve updating the model based on a relatively small new dataset, such as a few dozen or hundred annotated unipolar signals obtained during initial mapping procedures for the patient. The system may optionally incorporate regularization techniques, such as weight decay or dropout, during transfer learning to prevent overfitting to the small new dataset. Fine-tuning can be performed rapidly, enabling near real-time or same-session model adaptation during a clinical procedure.

Additionally, the system may include a transfer learning engine integrated into the console or optimization engine, which monitors incoming unipolar signals, identifies discrepancies or drift relative to expected signal patterns, and initiates transfer learning procedures automatically or under clinician supervision. By supporting transfer learning, the disclosed system enhances model generalizability and robustness, ensuring that far-field suppression remains accurate across diverse patient anatomies, disease states, and clinical conditions without the need for full model retraining.

In certain embodiments, the trained deep learning model for reducing far-field interference in unipolar cardiac signals is configured for deployment in both offline batch processing and real-time streaming analysis environments, depending on clinical requirements.

For offline batch processing, previously recorded unipolar signal datasets are processed after acquisition to suppress far-field components and enhance near-field signal clarity. In this mode, large volumes of unipolar signal data, such as those collected during an electrophysiological study or prior mapping session, can be input into the trained deep learning model in a batch format. The system processes the signals sequentially or in parallel, applying the far-field suppression model to each unipolar signal and generating processed outputs suitable for subsequent review, mapping, or diagnostic analysis. Offline batch processing is particularly useful for retrospective studies, research applications, or optimizing ablation strategies based on historical data.

For real-time streaming analysis, the trained deep learning model is integrated directly into the clinical workflow to process unipolar signals as they are acquired by the mapping catheter during an ongoing procedure. Incoming unipolar signals are streamed into the deep learning model, which applies the trained suppression algorithms with low-latency inference to output far-field-reduced signals in real time. These processed signals can be visualized on electroanatomical mapping systems, displayed alongside conventional unprocessed signals, and used to guide real-time decision-making during arrhythmia diagnosis and catheter ablation. In some embodiments, the system employs optimized hardware accelerators, such as GPUs or FPGAs, to meet the low-latency demands of real-time clinical environments.

The flexibility to operate in both offline and real-time modes ensures that the disclosed system can be deployed across a wide range of clinical use cases, from intraoperative guidance during catheter ablation procedures to post-procedural analysis and long-term patient monitoring studies. Furthermore, the system may allow clinicians to dynamically select between batch and streaming modes based on procedural needs, data availability, or specific mapping strategies.

In certain embodiments, the deep learning model for far-field suppression is deployed on a low-latency edge computing platform to enable real-time extraction of near-field components from unipolar cardiac signals. The edge computing platform may include dedicated hardware resources, such as embedded GPUs, field-programmable gate arrays (FPGAs), application-specific integrated circuits (ASICs), or optimized neural processing units (NPUs), which are located proximate to the data acquisition systems, such as within the catheter console, mapping system hardware, or adjacent auxiliary processors.

By executing the trained deep learning model on the edge computing platform, far-field interference can be suppressed immediately as unipolar signals are acquired, minimizing latency between data collection and output generation. This architecture reduces the computational burden placed on centralized clinical workstations and electrophysiology (EP) recording systems, which traditionally manage signal processing, display rendering, and procedural control tasks. Offloading the far-field suppression task to the edge device allows the primary clinical systems to operate more efficiently, maintaining responsiveness and avoiding potential bottlenecks during high-data-rate procedures such as high-density cardiac mapping or real-time ablation monitoring.

In some embodiments, the edge platform supports hardware-level parallelization and real-time inference optimizations, enabling the deep learning model to process multiple unipolar channels concurrently with minimal delay. The processed signals, containing enhanced near-field information, are then transmitted back to the clinical workstation or mapping system for visualization, mapping, or further analysis.

Additionally, the system may be configured such that the edge computing module can be modular, swappable, or integrated into the catheter system itself, thereby facilitating upgrades and future deployments of improved models without requiring replacement of core workstation infrastructure. In certain embodiments, the edge platform may also support periodic synchronization with centralized servers for model updates, logging, or clinical study data collection, while maintaining real-time operational independence during procedures.

Deploying the deep learning model on a low-latency edge computing platform thus ensures that near-field signal extraction is performed with minimal processing delay, enhances clinical workflow efficiency, and supports scalable, real-time electrophysiology mapping across diverse clinical environments.

The described embodiments have broad applications in fields that require precise localization and analysis of electrical activity within biological tissues or other systems. Its primary use lies in cardiac electrophysiology, where it can be employed to enhance the accuracy of cardiac mapping procedures. Specifically, the embodiments can be utilized in activation mapping to identify the sequence of electrical activation in the heart, voltage mapping to characterize myocardial tissue (e.g., detecting scarring or fibrosis), and arrhythmia diagnosis and treatment to pinpoint arrhythmic foci or reentrant circuits, guiding procedures such as catheter ablation. The embodiment's ability to extract near-field signals with high spatial and temporal precision makes it particularly valuable in treating complex arrhythmias like atrial fibrillation or ventricular tachycardia.

The described embodiments can significantly enhance precision and outcomes during cardiac surgery, particularly in procedures requiring real-time analysis of electrical activity, such as catheter ablation or pacemaker implantation. By accurately extracting near-field signals from unipolar recordings, the embodiment allows surgeons to localize arrhythmogenic sites with high precision, even in challenging environments where far-field signals or noise might otherwise obscure critical data.

During catheter ablation, the embodiments can be used to create detailed activation maps of the heart in real-time, enabling the identification of the exact origin of arrhythmias such as atrial fibrillation or ventricular tachycardia. Surgeons can use these maps to guide the ablation catheter to targeted tissue regions, minimizing damage to healthy tissue and increasing the procedure's success rate.

For implanting pacemakers or defibrillators, the embodiments ensure optimal electrode placement by providing precise measurements of local electrical activity at potential implantation sites. This helps ensure that the device effectively synchronizes the heart's rhythm, improving therapeutic efficacy.

Additionally, the embodiments can assist in surgical planning and intraoperative decision-making by integrating near-field signal data into 3D electroanatomical mapping systems. These maps provide a visual representation of conduction pathways and tissue properties, helping surgeons avoid critical structures and optimize surgical techniques.

In minimally invasive surgeries, where spatial and temporal precision is paramount, the embodiments can enhance outcomes by ensuring reliable signal extraction from unipolar recordings, even in conditions with limited access to multipolar systems. This capability reduces procedure time, enhances safety, and improves the overall accuracy of surgical interventions.

The results generated by the embodiments can be displayed during cardiac surgery using advanced visualization systems that integrate near-field signal data into real-time electroanatomical mapping tools. For example, near-field signal data can be processed into color-coded 3D maps that highlight regions of interest, such as arrhythmogenic sites, scar tissue, or areas with abnormal conduction, providing precise targets for catheter ablation. Real-time wave propagation patterns may be displayed as animations overlaid on anatomical heart images, helping to identify reentrant circuits or focal arrhythmia sources by illustrating the direction and speed of electrical activation. Additionally, near-field signal waveforms can be presented alongside traditional ECG-like tracings to emphasize sharp, localized signals relevant to the surgical site, assisting in monitoring tissue viability and assessing the effects of ablation. These results can also be incorporated into augmented reality (AR) or virtual reality (VR) systems, projecting activation maps and signal data directly onto the surgeon's view of the heart, enhancing guidance without distraction. Integrated monitors may combine live near-field signal overlays with fluoroscopic images, hemodynamic data, and catheter positions to provide a comprehensive view for the surgical team, while touchscreen mapping systems could allow surgeons to interact directly with the data, zooming into specific regions, highlighting areas of interest, or simulating interventions. These display methods ensure that extracted near-field signals are presented in an intuitive and actionable format, enhancing surgical precision and decision-making

Beyond cardiac applications, the embodiments can be used in neurophysiology, where precise signal extraction from unipolar neural recordings is essential for studying brain activity, diagnosing neurological disorders, or developing brain-computer interfaces. It may also be applied in bioelectric signal processing for other organ systems, such as in gastrointestinal or muscular studies, where electrical signal mapping aids in diagnosing functional abnormalities. Additionally, the embodiments have potential in non-biological contexts, such as industrial systems that rely on electrical signal analysis for fault detection or signal quality assessment. In all these domains, the embodiments provide a scalable, high-accuracy solution for extracting localized signal information from unipolar recordings, even in environments where multipolar systems are unavailable.

The disclosed methods provide several advantages over the existing Multipolar algorithm. While the traditional approach requires multiple unipolar signals to compute a single multipolar signal, the deep learning model requires only a single unipolar signal as input. The current algorithm is also restricted to use with specific catheters, such as the OPTRELL^{™} catheter, whereas the deep learning model can be applied to different catheter designs with similar electrode characteristics. Additionally, the Multipolar algorithm's performance degrades when processing unipolar signals recorded during atrial fibrillation, whereas the deep learning model can improve performance due to its ability to learn complex signal relationships from diverse training data. Moreover, the Multipolar algorithm's effectiveness is limited by electrode placement and catheter geometry, whereas the deep learning model is not constrained by such factors, making it more robust across different recording conditions.

To implement this method, a sufficiently diverse training dataset must be compiled to ensure the deep learning model generalizes across different types of unipolar signals. The training process requires computational resources such as GPUs for efficient optimization, though once trained, the model can operate in real-time on standard electrophysiology hardware. Given the clinical applications, the model may require validation and regulatory approval before deployment in medical settings.

By addressing the limitations of conventional far-field removal techniques, the present invention offers an efficient and adaptable solution for improving the quality of electrophysiological signal analysis. This deep learning approach enhances the accuracy of unipolar signal processing, enabling broader adoption across various electrophysiological mapping systems while improving signal fidelity in complex cardiac conditions.

In an aspect, training and applying a machine learning model, as described in reference to FIGS. 3, and 5-7, may be performed by the systems described herein, 100 (FIG. 1) or 200 (FIG. 2), also representing a Mapping System, in real-time, on a server at the facility where the cardiac procedure is taking place, such as a hospital or medical facility, or at a remote location, such as in the cloud or at a training center.

Although features and elements are described above in particular combinations, one of ordinary skill in the art will appreciate that each feature or element can be used alone or in any combination with the other features and elements. In addition, although process steps are described above in a particular order, the steps can be performed in other desirable orders.

The methods, processes, modules, and systems described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, read only memory (ROM), random access memory (RAM), a register, cache memory, semiconductor memory devices, magnetic media such as internal hard disks and removable disks, magneto-optical media, and optical media such as CD-ROM disks, and digital versatile disks (DVDs). A processor in association with software may be used to implement a radio frequency transceiver for use in a WTRU, UE, terminal, base station, RNC, or any host computer.

Further embodiments herein may be formed by supplementing an embodiment with one or more element from any one or more other embodiment herein, and/or substituting one or more element from one embodiment with one or more element from one or more other embodiment herein.

It is understood, therefore, that the disclosed subject matter is not limited to the particular embodiments disclosed, but is intended to cover all modifications which are within the spirit and scope of the disclosure as defined by the appended claims, the above description, and/or shown in the attached drawings.

Aspects of the invention:
1. A method for real-time far-field reduction in unipolar cardiac signals, the method comprising:
   generating training labels by applying a multipolar algorithm to unipolar signals, thereby creating multipolar signals with reduced far-field effects;
   training a deep learning model using the training labels;
   deploying the trained deep learning model to receive a single unipolar signal as input and output a processed signal with far-field interference suppressed; and
   using the processed signal for real-time cardiac mapping, arrhythmia detection, or catheter ablation guidance.
2. The method of aspect 1, wherein the real-time far-field reduction is performed using edge computing or cloud-based processing to enable high-speed cardiac analysis.
3. The method of aspect 1, wherein the processed signal is used to automate detection of arrhythmogenic sites.
4. The method of aspect 1, wherein the trained deep learning model is deployed for offline batch processing or real-time streaming analysis in clinical settings.
5. The method of aspect 1, further comprising:
   displaying the processed signal during catheter ablation procedures to assist in accurate lesion placement by reducing far-field distortion.
6. The method of aspect 1, wherein the trained deep learning model is periodically updated using incremental learning or federated learning, allowing continuous performance improvements based on new electrophysiological recordings without requiring complete retraining on a centralized dataset.

## Claims

1. A method for reducing far-field interference in unipolar cardiac signals, comprising:
receiving multiple unipolar signals, including a center unipolar signal and additional unipolar signals recorded from a cardiac mapping catheter;
applying a multipolar algorithm to estimate and remove far-field components from the center unipolar signal, thereby generating a multipolar signal; and
training a deep learning model using the multipolar signal as a ground truth label to form a trained deep learning model;
optimizing the trained deep learning model to learn near-field signal extraction by minimizing the far-field interference; and
applying the trained deep learning model to new unipolar signals to generate a processed output with reduced far-field effects, wherein the trained deep learning model applies a single new unipolar signal as input.

2. The method of claim 1, further comprising:
applying bandpass filtering, wavelet transformation, or adaptive noise filtering to the multipolar signal.

3. The method of claim 1, wherein the deep learning model comprises a convolutional neural network (CNN) combined with a recurrent neural network (RNN) to process both spatial and temporal signal features.

4. The method of claim 1, wherein the deep learning model is optimized using a loss function based on mean squared error (MSE), mean absolute error (MAE), or custom signal reconstruction metrics.

5. The method of claim 1, wherein the deep learning model is trained using data augmentation techniques, including time shifting, amplitude scaling, or synthetic noise addition, to enhance model generalization.

6. The method of claim 1, wherein the deep learning model further incorporates domain adaptation techniques to enhance performance across different catheter types and electrode configurations.

7. A system for far-field suppression in unipolar cardiac signals, the system comprising:
a memory; and
one or more processors that are communicatively coupled to the memory,
wherein the one or more processors are collectively configured to:
receive multiple unipolar signals and apply a multipolar algorithm to generate far-field-reduced multipolar signals as ground truth labels;
train a deep learning model using the ground truth labels;
form processed signals by applying the trained deep learning model to new unipolar signals to extract near-field components and suppress far-field interference; and
provide the processed signals for electrophysiological analysis, real-time mapping, or integration with cardiac diagnostic systems.

8. The system of claim 7, wherein the one or more processors are collectively configured to operate with multiple catheter types, enabling cross-platform compatibility.

9. The system of claim 7, wherein the one or more processors are further configured to:
integrate an attention mechanism to improve detection of near-field components while suppressing far-field interference.

10. The system of claim 7, wherein the one or more processors are further collectively configured to:
display a 3D electroanatomical mapping interface that visualizes the processed signals.

11. The system of claim 7, wherein the deep learning model supports transfer learning, allowing the deep learning model to adapt to new patient data with minimal retraining.

12. The system of claim 7, wherein the deep learning model continuously improves through adaptive learning, updating model parameters based on new patient data collected during electrophysiological procedures.

13. The system of claim 7, wherein the deep learning model is deployed on a low-latency edge computing platform to enable real-time near-field signal extraction, reducing computational burden on clinical workstations and electrophysiology recording systems.
